# EUROPÄISCHE PATENTSCHRIFT

(11) **EP 2 308 455 B1**
(45) Veröffentlichungstag und Bekanntmachung des Hinweises auf die Patenterteilung: **03.01.2018**
(21) Anmeldenummer: 10171444.2
(22) Anmeldetag: 30.07.2010
(51) Int. Cl.: A61K 8/04, A61K 8/22, A61Q 5/10, A61K 8/44, A61K 8/60

(54) **Schaumförmige Färbemittel II**
Foaming dye II
Colorant II sous forme de mousse

(30) Priorität: 13.08.2009 DE 102009028523
(43) Veröffentlichungstag der Anmeldung: 13.04.2011
(73) Patentinhaber: Henkel AG & Co. KGaA, 40589 Düsseldorf (DE)
(72) Erfinder: Kleen, Astrid, 20457 Hamburg (DE); Förster, Thomas, 40591 Düsseldorf (DE); Zirwen, Sabrina, 22089 Hamburg (DE); Akram, Mustafa, 22455 Hamburg (DE); Delli Venneri, Nicola, 40667 Meerbusch (IT)

(56) Entgegenhaltungen:
- EP-A1- 1 470 812
- EP-A1- 1 557 157
- WO-A1-2006/012935

## Beschreibung

Die Erfindung betrifft Mittel zum Färben, die geeignet sind, durch spezielle Applikationsvorrichtungen in Form eines stabilen Schaums ausgetragen zu werden, ein Färbeverfahren unter Zuhilfenahme der Mittel und der Applikationsvorrichtung sowie ein entsprechendes Kit zur Färbung von keratinhaltigen Fasern.

Für das Färben von keratinhaltigen Fasern kommen im allgemeinen entweder direktziehende Farbstoffe oder Oxidationsfarbstoffe, die durch oxidative Kupplung einer oder mehrerer Entwicklerkomponenten untereinander oder mit einer oder mehreren Kupplerkomponenten entstehen, zur Anwendung. Kuppler- und Entwicklerkomponenten werden auch als Oxidationsfarbstoffvorprodukte bezeichnet.

Oxidative Färbemittel bestehen üblicherweise aus zwei Komponenten, deren Mischung ausreichend zähflüssig ist, damit sie bequem auf die Haare aufgetragen werden kann und dabei nicht tropfen oder verlaufen. Darüber hinaus gab es häufig Versuche, andere Konfektionierungsformen zu entwickeln. So wurde vorgeschlagen, dünnflüssigere Färbemittel mit speziellen Applikatorsystemen auf die Haare aufzubringen oder Färbemittel als Schaum zu applizieren. Bei der Schaumapplikation ist insbesondere der Einsatz von Aerosolschäumen verbreitet. Allerdings besteht in jüngerer Zeit auch das Bedürfnis auf den Einsatz von Treibgasen verzichten zu können.

Die europäische Patentanmeldung EP 1 470 812 A1 schlägt dazu vor, zwei Agentien bereitzustellen, von denen eines Alkalisierungsmittel und das andere Wasserstoffperoxid enthält, wobei mindestens eines der Mittel Tenside enthält, und diese beiden Mittel miteinander zu vermischen und aus einem Schaumapplikator in Form eines Schaumes auszubringen.

Bei Oxidationsfarben für keratinische Fasern müssen Farbstoffvorprodukte und Oxidationsmittel zunächst getrennt vorliegen, um eine vorzeitige Reaktion zu verhindern. Ein höherer Tensidgehalt in der (üblicherweise alkalischen) Mischung der Farbstoffvorprodukte ist nicht immer erwünscht, da ein Aufschäumen bei der Herstellung und beim Transport auftreten kann und die Innenseite der (üblicherweise durchsichtigen) Verpackung dann für den Verbraucher unansehnlich wirkt. Auch ein Tensidgehalt in der Wasserstoffperoxidlösung kann problematisch sein, da ein Auf- und

Überschäumen bei Mischung mit den Reaktionskomponenten auftreten kann. Der Verbraucher kann dann ggf. den Applikatorkopf nicht schnell genug auf die Flasche, in der die Komponenten gemischt werden, aufschrauben, und es tritt ein Überschäumen und eine Verschmutzung der Arbeitsumgebung auf.

Ein weiteres Problem bei der Schaumapplikation ist die Stabilisierung der Schäume. Die Beschaffenheit von Schäumen ist dann als ideal zu bezeichnen, wenn bei der Produktabgabe ein fester, stabiler Schaum entsteht, der ein geschmeidiges Gefühl hinterlässt und auf dem Haar nur langsam bricht. Häufig ist aber zu beobachten, dass die ausgebrachten Schäume wenig stabil sind und schnell wieder in sich zusammenfallen, so dass eine dünnflüssige, tropfende Lösung zurückbleibt. Andererseits ist es auch wesentlich, dass der Schaum trotzdem die Haare gut benetzt, so dass ein guter Farbauftrag stattfindet kann.

Aufgabe der vorliegenden Erfindung war es daher, Färbemittel für die Schaumapplikation, insbesondere ohne Verwendung von Treibgasen, zu optimieren, so dass die oben genannten Nachteile überwunden werden können.

Es wurde nun überraschenderweise gefunden, daß sich Oxidationsmittel und Farbstoffvorprodukte problemlos und ohne Auf- und Überschäumen mischen lassen, wenn die entsprechenden Zubereitungen tensidfrei formuliert werden. Die nachträgliche Zugabe von Tensid führt einerseits zu einem verzögerten Aufschäumen durch das Mischen und somit zu genügend Zeit, einen sauberen Behälter dicht zu verschließen, und andererseits zu stabileren und applikationsreundlicheren Schäumen.

Gegenstand der Erfindung ist in einer ersten Ausführungsform ein Haarbehandlungs-Kit, umfassend mindestens drei getrennt voneinander konfektionierte Mittel A, B und C, wobei
a. das erste Mittel (A) tensidfrei ist und mindestens ein Oxidationsfarbstoffvorprodukt enthält und
b. das zweite Mittel (B) tensidfrei ist und mindestens ein Oxidationsmittel enthält und
c. das dritte Mittel (C) mindestens ein Tensid enthält und
d. mindestens eines der Mittel A, B oder C in einer Applikationsvorrichtung enthalten ist, die geeignet ist, die Mittel A, B und C miteinander zu mischen und die Mischung in Form eines Schaumes abzugeben,
wobei die Applikationsvorrichtung ein Pumpschäumer oder ein Squeezeschäumer ist.

Das erfindungsgemäße Haarbehandlungs-Kit ist insbesondere zur Färbung keratinischer Fasern geeignet und bestimmt. Unter keratinhaltigen Fasern sind Wolle, Pelze, Federn und insbesondere menschliche Haare zu verstehen. Die erfindungsgemäßen Färbemittel können prinzipiell aber auch zum Färben anderer Naturfasern, wie z. B. Baumwolle, Jute, Sisal, Leinen oder Seide, modifizierter Naturfasern, wie beispielsweise Regeneratcellulose, Nitro-, Alkyl- oder Hydroxyalkyl- oder Acetylcellulose verwendet werden.

Die erfindungsgemäßen Kits enthalten mindestens drei getrennt voneinander konfektionierte Mittel A, B und C. "Konfektioniert" bedeutet im Rahmen der vorliegenden Erfindung hergestellt und verkaufsbereit verpackt, d.h. die erfindungsgemäßen Kits enthalten drei Zubereitungen (= Mittel), die getrennt voneinander verpackt im erfindungsgemäßen Kit vorliegen.

Das erste Mittel (A) ist tensidfrei und enthält mindestens ein Oxidationsfarbstoffvorprodukt. "Tensidfrei" bedeutet im Rahmen der vorliegenden Erfindung, daß das betreffende Mittel bezogen auf sein Gewicht weniger als 1 Gew.-% Tensid(e), vorzugsweise weniger als 0,5 Gew.-%, weiter bevorzugt weniger als 0,25 Gew.-% und insbesondere weniger als 0,1 Gew.-% Tensid(e) enthält. Ganz besonders bevorzugt bedeutet "tensidfrei" im Rahmen der vorliegenden Erfindung, daß das betreffende Mittel weniger als 0,001 Gew.-% Tensid(e) enthält.

Als erste erfindungswesentliche Komponente enthalten die Mittel A mindestens ein Oxidationsfarbstoffvorprodukt. Vorzugsweise enthält das Mittel A eine oder mehrere Entwicklerkomponenten sowie gegebenenfalls eine oder mehrere Kupplerkomponenten.

Die Entwicklerkomponenten bilden unter dem Einfluß von Oxidationsmitteln oder von Luftsauerstoff untereinander oder unter Kupplung mit einer oder mehreren Kupplerkomponenten die eigentlichen Farbstoffe aus.

Es kann erfindungsgemäß bevorzugt sein, als Entwicklerkomponente ein p-Phenylendiaminderivat oder eines seiner physiologisch verträglichen Salze einzusetzen.
Besonders bevorzugte p-Phenylendiamine sind ausgewählt aus p-Phenylendiamin, p-Toluylendiamin, 2-Chlor-p-phenylendiamin, N,N-Bis-(2-hydroxyethyl)-p-phenylendiamin und N-Phenyl-p-phenylendiamin, 2-(2-Hydroxyethyl)-p-phenylendiamin, sowie ihren physiologisch verträglichen Salzen.

Es kann erfindungsgemäß weiterhin bevorzugt sein, als Entwicklerkomponente Verbindungen einzusetzen, die mindestens zwei aromatische Kerne enthalten, die mit Amino- und/oder Hydroxylgruppen substituiert sind.
Bevorzugte zweikernige Entwicklerkomponenten sind insbesondere: N,N'-Bis-(2-hydroxyethyl)-N,N'-bis-(4'-aminophenyl)-1,3-diamino-propan-2-ol und Bis-(2-hydroxy-5-aminophenyl)-methan und deren physiologisch verträglichen Salze.

Weiterhin kann es erfindungsgemäß bevorzugt sein, als Entwicklerkomponente ein p-Aminophenolderivat oder eines seiner physiologisch verträglichen Salze einzusetzen.

Bevorzugte p-Aminophenole sind insbesondere p-Aminophenol, N-Methyl-p-aminophenol, und 4-Amino-3-methyl-phenol sowie deren physiologisch verträglichen Salze.

Ferner kann die Entwicklerkomponente ausgewählt sein aus o-Aminophenol und seinen Derivaten, wie beispielsweise 2-Amino-5-methylphenol und dessen physiologisch verträglichen Salze.

Weiterhin kann die Entwicklerkomponente ausgewählt sein aus heterocyclischen Entwicklerkomponenten, wie beispielsweise den Pyridin-, Pyrimidin-, Pyrazol-, Pyrazol-PyrimidinDerivaten und ihren physiologisch verträglichen Salzen.

Bevorzugte Pyrimidin-Derivate sind insbesondere 2,4,5,6-Tetraaminopyrimidin und 4-Hydroxy-2,5,6-triaminopyrimidin und deren physiologisch verträglichen Salze.

Ein bevorzugtes Pyrazol-Derivat ist 4,5-Diamino-1-(2-hydroxyethyl)-pyrazol und dessen physiologisch verträglichen Salze.

Die Entwicklerkomponenten werden bevorzugt in einer Menge von 0,005 bis 20 Gew.-%, vorzugsweise 0,1 bis 5 Gew.-%, jeweils bezogen auf das anwendungsbereite Oxidationsfärbemittel, d.h. die Mischung der Komponenten A, B und C verwendet. Die Mengen an Entwicklerkomponenten, bezogen auf das Mittel A, sind dementsprechend vorzugsweise höher.

Kupplerkomponenten bilden im Rahmen der oxidativen Färbung allein keine signifikante Färbung aus, sondern benötigen stets die Gegenwart von Entwicklerkomponenten. Daher ist es erfindungsgemäß bevorzugt, dass bei Verwendung mindestens einer Entwicklerkomponente zusätzlich mindestens eine Kupplerkomponente zum Einsatz kommt.

Kupplerkomponenten im Sinne der Erfindung erlauben mindestens eine Substitution eines chemischen Restes des Kupplers durch die oxidierte Form der Entwicklerkomponente. Dabei bildet sich eine kovalente Bindung zwischen Kuppler- und Entwicklerkomponente aus. Kuppler sind bevorzugt zyklische Verbindungen, die am Zyklus mindestens zwei Gruppen tragen, ausgewählt aus (i) gegebenenfalls substituierten Aminogruppen und/oder (ii) Hydroxygruppen. Diese Gruppen stehen durch ein Doppelbindungssystem in Konjugation. Wenn die zyklische Verbindung ein Sechsring ist, so befinden sich die besagten Gruppen bevorzugt in ortho-Position oder meta-Position zueinander.

Erfindungsgemäße Kupplerkomponenten werden bevorzugt als mindestens eine Verbindung aus einer der folgenden Klassen ausgewählt:
- m-Aminophenol und/oder dessen Derivate,
- m-Diaminobenzol und/oder dessen Derivate,
- o-Diaminobenzol und/oder dessen Derivate,
- Naphthalinderivate mit mindestens einer Hydroxygruppe,
- Di- beziehungsweise Trihydroxybenzol und/oder deren Derivate,
- Pyridinderivate,
- Pyrimidinderivate,
- Monohydroxyindol-Derivate und/oder Monoaminoindol-Derivate,
- Monohydroxyindolin-Derivate und/oder Monoaminoindolin-Derivate,
- Pyrazolonderivate, wie beispielsweise 1-Phenyl-3-methylpyrazol-5-on,
- Morpholinderivate wie beispielsweise 6-Hydroxybenzomorpholin oder 6-Amino-benzomorpholin,
- Chinoxalinderivate wie beispielsweise 6-Methyl-1,2,3,4-tetrahydrochinoxalin,

Gemische aus zwei oder mehrer Verbindungen aus einer oder mehrerer dieser Klassen sind im Rahmen dieser Ausführungsform ebenso erfindungsgemäß.

Als Kupplersubstanzen eignen sich insbesondere 1-Naphthol, 1,5- und 2,7-Dihydroxynaphthalin, 1-Acetoxy-2-methoxynaphthalin, Resorcin, 4-Chlor-resorcin und 2-Amino-3-hydroxypyridin und deren physiologisch verträglichen Salze.

Erfindungsgemäß bevorzugte Kupplerkomponenten sind
(A) m-Aminophenol und dessen Derivate wie beispielsweise 5-Amino-2-methylphenol, 3-Amino-2-chlor-6-methylphenol, 5-Amino-4-chlor-2-methylphenol, 5-(2'-Hydroxyethyl)-amino-2-methylphenol und 2,4-Dichlor-3-aminophenol,
(B) o-Aminophenol und dessen Derivate, beispielsweise 2-Amino-5-ethylphenol,
(C) m-Diaminobenzol und dessen Derivate wie beispielsweise 2,4-Diaminophenoxy-ethanol, 1,3-Bis-(2',4'-diaminophenoxy)-propan, 1-Methoxy-2-amino-4-(2'-hydroxyethylamino)benzol, 2,6-Bis-(2'-hydroxyethylamino)-1-methylbenzol,
(D) o-Diaminobenzol und dessen Derivate,
(E) Di- beziehungsweise Trihydroxybenzolderivate wie beispielsweise 2-Methylresorcin und 1,2,4-Trihydroxybenzol,
(F) Pyridinderivate wie beispielsweise 3-Amino-2-methylamino-6-methoxypyridin, 2,6-Diaminopyridin, 2,6-Dihydroxy-3,4-dimethylpyridin und 3,5-Diamino-2,6-dimethoxypyridin,
(G) Naphthalinderivate wie beispielsweise 1-Naphthol und 2-Methyl-1-naphthol,
(H) Morpholinderivate wie beispielsweise 6-Hydroxybenzomorpholin,
(I) Chinoxalinderivate,
(J) Pyrazolderivate wie beispielsweise 1-Phenyl-3-methylpyrazol-5-on,
(K) Indolderivate wie beispielsweise 6-Hydroxyindol,
(L) Pyrimidinderivate oder
(M) Methylendioxybenzolderivate wie beispielsweise 1-(2'-Hydroxyethyl)-amino-3,4-methylendioxybenzol
sowie deren physiologisch verträglichen Salze.

Erfindungsgemäß besonders bevorzugte Kupplerkomponenten sind 1-Naphthol, 1,5- und 2,7-Dihydroxynaphthalin, 5-Amino-2-methylphenol, 2-Amino-3-hydroxypyridin, Resorcin, 4-Chlorresorcin, 2-Methylresorcin und 2,6-Dihydroxy-3,4-dimethylpyridin und deren physiologisch verträglichen Salze.

Die Kupplerkomponenten werden bevorzugt in einer Menge von 0,005 bis 20 Gew.-%, vorzugsweise 0,1 bis 5 Gew.-%, jeweils bezogen auf das anwendungsbereite Oxidationsfärbemittel, d.h. die Mischung der Komponenten A, B und C verwendet. Die Mengen an Kupplerkomponenten, bezogen auf das Mittel A, sind dementsprechend vorzugsweise höher.

Dabei werden Entwicklerkomponenten und Kupplerkomponenten im Allgemeinen in etwa molaren Mengen zueinander eingesetzt. Wenn sich auch der molare Einsatz als zweckmäßig erwiesen hat, so ist ein gewisser Überschuss einzelner Oxidationsfarbstoffvorprodukte nicht nachteilig, so dass Entwicklerkomponenten und Kupplerkomponenten in einem Mol-Verhältnis von 1:0,5 bis 1:3, insbesondere 1:1 bis 1:2, stehen können.

Zusammenfassend sind erfindungsgemäße Haarbehandlungs-Kits bevorzugt, bei denen das erste Mittel (A), bezogen auf das Gewicht des Mittels A, 0,1 bis 20 Gew.-%, vorzugsweise 0,25 bis 17,5 Gew.-%, weiter bevorzugt 0,5 bis 15 und insbesondere 0,75 bis 10 Gew.-% Oxidationsfarbstoffvorprodukt(e) enthält.

Zur weiteren Nuancierung der resultierenden Farbtöne kann es erfindungsgemäß bevorzugt sein, den Mittel weiterhin mindestens einen direktziehenden Farbstoff zuzusetzen. Hierbei handelt es sich um Farbstoffmoleküle, die direkt auf das Substrat aufziehen und keinen oxidativen Prozeß zur Ausbildung der Farbe benötigen. Zu diesen Farbstoffen gehört beispielsweise das bereits aus dem Altertum zur Färbung von Körper und Haaren bekannte Henna.
Heutzutage sind direktziehende Farbstoffe üblicherweise Nitrophenylendiamine, Nitroaminophenole, Azofarbstoffe, Anthrachinone oder Indophenole.

Die direktziehenden Farbstoffe werden jeweils bevorzugt in einer Menge von 0,001 bis 20 Gew.-%, bezogen auf die gesamte Anwendungszubereitung, eingesetzt. Die Gesamtmenge an direktziehenden Farbstoffen beträgt vorzugsweise höchstens 20 Gew.-%. Erfindungsgemäß bevorzugte Haarbehandlungs-Kits sind dadurch gekennzeichnet, daß das erste Mittel (A) zusätzlich mindestens einen direktziehenden Farbstoff enthält.

Direktziehende Farbstoffe können in anionische, kationische und nichtionische direktziehende Farbstoffe unterteilt werden.

Als anionische direktziehende Farbstoffe eignen sich insbesondere 2,4-Dinitro-1-naphthol-7-sulfonsäure-dinatriumsalz (C.I. 10,316; Acid Yellow 1; Food Yellow No. 1), 2-(Indan-1,3-dion-2-yl)chinolin-x,x-sulfonsäure (Gemisch aus Mono- und Disulfonsäure) (C.I. 47,005; D&C Yellow No. 10; Food Yellow No. 13; Acid Yellow 3, Yellow 10), 5-Hydroxy-1-(4-sulfophenyl)-4-[(4-sulfophenyl)-azo]pyrazol-3-carbonsäure-trinatriumsalz (C.I. 19,140; Food Yellow No. 4; Acid Yellow 23), 3-[(4-Phenylamino)phenyl]azobezolsulfonsäuresäure-natriumsalz (C.I. 13,065; Ki406; Acid Yellow 36), 4-[(2-Hydroxynaphth-1-yl)azo]-benzolsulfonsäure-natriumsalz (C.I. 15,510; Acid Orange 7), 6-Hydroxy-5-[(4-sulfonaphth-1-yl)azo]-2,4-naphthalin-disulfonsäure-trinatriumsalz (C.I. 16,255; Ponceau 4R; Acid Red 18), 8-Amino-1-hydroxy-2-(phenylazo)-3,6-naphthalin-disulfonsäure-dinatriumsalz (C.I. 17,200; Acid Red 33; Red 33), N-[6-(Diethylamino)-9-(2,4-disulfophenyl)-3H-xanthen-3-yliden]-N-ethylethanammonium-hydroxid, inneres Salz, Natriumsalz (C.I. 45,100; Acid Red 52), 2',4',5',7'-Tetrabrom-4,5,6,7-tetrachlor-3',6'- dihydroxyspiro[isobenzofuran-1(3H),9'[9H]xanthen]-3-on-dinatriumsalz (C.I. 45,410; Acid Red 92), 3-Hydroxy-4-[(4-methyl-2-sulfonphenyl)azo]-2-naphthalincarbonsäure-calciumsalz (C.I. 15,850:1; Pigment Red 57:1), 1,4-Bis[(2-sulfo-4-methylphenyl)amino]-9,10-anthrachinon-dinatriumsalz (C.I. 61,570; Acid Green 25), Bis[4-(dimethylamino)phenyl]-(3,7-disulfo-2-hydroxynaphth-1-yl)carbenium-inneres Salz, Natriumsalz (C.I. 44,090; Food Green No. 4; Acid Green 50), N-[4-[(2,4-Disulfophenyl)[4-[ethyl(phenylmethyl)amino)phenyl]methylen]-2,5-cyclohexadien-1-yliden]-N-ethylbenzolmethanaminium-hydroxid, inneres Salz, Natriumsalz (C.I. 42,080; Acid Blue 7), (2-Sulfophenyl)di[4-(ethyl((4-sulfophenyl)methyl)amino)phenyl]-carbenium-dinatriumsalz Betain (C.I. 42,090; Acid Blue 9; FD&C Blue No. 1), 1-Amino-4-(cyclohexylamino)-9,10-anthrachinon-2-sulfonsäure-natriumsalz (C.I. 62045; Acid Blue 62), 1-Hydroxy-4-[(4-methyl-2- sulfophenyl)amino]-9,10-anthrachinon-natriumsalz (C.I. 60,730; D&C Violett No. 2; Acid Violet 43), 5-Amino-4-hydroxy-6-[(4-nitrophenyl)-azo]-3-(phenylazo)-2,7-naphthalin-disulfonsäure-dinatriumsalz (C.I. 20,470; Acid Black 1), 3-Hydroxy-4-[(2-hydroxynaphth-1-yl)azo]-7-nitro-1-naphthalin-sulfonsäure-chromkomplex (3:2) (C.I. 15,711; Acid Black 52), 3',3",4,5,5',5",6,7-Octabromphenolsulfonphthalein (Tetrabromphenolblau).

Bevorzugte anionische direktziehende Farbstoffe sind die unter den internationalen Bezeichnungen bzw. Handelsnamen Acid Yellow 1, Acid Yellow 10, Acid Yellow 23, Acid Yellow 36, Acid Orange 7, Acid Red 33, Acid Red 52, Pigment Red 57:1, Acid Blue 7, Acid Green 50, Acid Violet 43, Acid Black 1 und Acid Black 52 bekannten Verbindungen.

Als kationische direktziehende Farbstoffe eignen sich insbesondere Di[4-(diethylamino)phenyl][4-(ethylamino)naphthyl]carbenium-chlorid (C.I. 42,595; Basic Blue 7), Di[4-(dimethylamino)phenyl][4-(phenylamino)naphthyl] carbenium-chlorid (C.I. 44,045; Basic Blue 26), 8-Amino-2-brom-5-hydroxy-4-imino-6-[(3-(trimethylammonio)phenyl)amino]-1(4H)-naphthalinon-chlorid (C.I. 56,059; Basic Blue No. 99), Tri(4-amino-3-methylphenyl)carbenium-chlorid (C.I. 42,520; Basic Violet 2), Di(4-aminophenyl)(4-amino-3- methylphenyl)carbeniumchlorid (C.I. 42,510 Basic Violet 14), 1-[(4-Aminophenyl)azo]-7-(trimethylammonio)- 2-naphthol-chlorid (C.I. 12,250; Basic Brown 16), 1-[(4-Amino-3-nitrophenyl)azo]-7-(trimethylammonio)-2-naphthol-chlorid (C.I. 12,251; Basic Brown 17), 3-[(4-Amino-2,5- dimethoxyphenyl)azo]-N,N,N-trimethylbenzolaminium-chlorid (C.I. 12,605, Basic Orange 69), 2-[((4-Dimethylamino)phenyl)azo]-1,3-dimethyl-1H-imidazoliumchlorid (Basic Red 51), 2-Hydroxy-1-[(2-methoxyphenyl)azo]-7-(trimethylammonio)-naphthalin-chlorid (C.I. 12,245; Basic Red 76), 2-[4-Aminophenyl]azo]-1,3-dimethyl-1H-Imidazolium-chlorid (Basic Orange 31), 3-Methyl-1-phenyl-4-[(3-(trimethylammonio)phenyl)azo]-pyrazol-5-on-chlorid (C.I. 12,719; Basic Yellow 57), 1-Methyl-4-((methylphenylhydrazono)methyl)-pyridinium-methylsulfat (Basic Yellow 87), 1-(2-Morpholiniumpropylamino)-4-hydroxy-9,10-anthrachinon-methylsulfat, 4-Formyl-1-methylquinolonium-p-toluensulfonat und direktziehende Farbstoffe, die einen Heterocyclus enthalten, der mindestens ein quaternäres Stickstoffatom aufweist.

Als nichtionische direktziehende Farbstoffe eignen sich insbesondere nichtionische Nitro- und Chinonfarbstoffe und neutrale Azofarbstoffe.

Geeignete blaue Nitrofarbstoffe sind insbesondere 1,4-Bis[(2-hydroxyethyl)amino]-2-nitrobenzol (HC Violet BS), 1-(2-Hydroxyethyl)amino-2-nitro-4-[di(2-hydroxyethyl)amino]-benzol (HC Blue 2), 4-[Di(2-hydroxyethyl)amino]-1-[(2-methoxyethyl)amino]-2-nitrobenzol (HC Blue 11), 4-[Ethyl-(2-hydroxyethyl)-amino]-1-[(2-hydroxyethyl)amino]-2-nitrobenzol-hydrochlorid (HC Blue 12), 1-(2-Hydroxyethyl)amino-2-nitro-4-N-ethyl-N-(2-hydroxyethyl)aminobenzol (HC Blue 15), 1-Amino-3-methyl-4-[(2-hydroxyethyl)amino]-6-nitrobenzol (HC Violet 1), 1-(3-Hydroxypropylamino)-4-[di(2-hydroxyethyl)amino]-2-nitrobenzol (HC Violet 2).

Geeignete rote Nitrofarbstoffe sind insbesondere 1-Amino-4-[(2-hydroxyethyl)amino]-2-nitrobenzol (HC Red 7), 2-Amino-4,6-dinitrophenol (Pikraminsäure) und deren Salze, 1,4-Diamino-2-nitrobenzol (C.I. 76,070), 4-Amino-2-nitro-diphenylamin (HC Red 1),), 1-Amino-4-[di(2-hydroxyethyl)amino]-2-nitrobenzol-hydrochlorid (HC Red 13), 1-Amino-4-[(2-hydroxyethyl)-amino]-5-chlor-2-nitrobenzol, 4-Amino-1-[(2-hydroxyethyl)amino]-2-nitrobenzol (HC Red 3), 4-[(2-Hydroxyethyl)-amino]-3-nitrotoluol, 4-Amino-3-nitrophenol, 4-[(2-Hydroxyethyl)-amino]-3-nitrophenol, 4-[(2-Nitrophenyl)amino]phenol (HC Orange 1), 1-[(2-Aminoethyl)amino]-4-(2-hydroxyethoxy)-2-nitrobenzol (HC Orange 2), 1-Amino-5-chlor-4-[(2,3-dihydroxypropyl)amino]-2-nitrobenzol (HC Red 10), 5-Chlor-1,4-[di(2,3-dihydroxypropyl)amino]-2-nitrobenzol (HC Red 11), 2-[(2- Hydroxyethyl)amino]-4,6-dinitrophenol und deren Salze, 4-Ethylamino-3-nitrobenzoesäure, 2-[(4-Amino-2-nitrophenyl)amino]-benzoesäure, 2-Chlor-6-ethylamino-4-nitrophenol, 2-Amino-6-chlor-4-nitrophenol, 4-[(3-Hydroxypropyl)amino]-3-nitrophenol (HC Red BN), 1,2,3,4-Tetrahydro-6-nitrochinoxalin, 6-Hydroxy-5-((2-methoxy-5-methyl-4-sulfophenyl)azo)- 2-naphthalensulfonsäure (Curry Red).

Geeignete gelbe Nitrofarbstoffe sind insbesondere 1,2-Diamino-4-nitrobenzol (C.I. 76,020), 1-[(2-Hydroxyethyl)amino]-2-nitrobenzol (HC Yellow 2), 1-(2-Hydroxyethoxy)-2-[(2-hydroxyethyl)amino]-5-nitrobenzol (HC Yellow 4), 1-Amino-2-[(2-hydroxyethyl)amino]-5-nitrobenzol (HC Yellow 5), 4-[(2,3-Dihydroxypropyl)-amino]-3-nitro-1-trifluormethyl-benzol (HC Yellow 6), 2-[(2- Hydroxyethyl)-amino]-1-methoxy-5-nitrobenzol, 2-Amino-4-nitrophenol, 1-(2-Hydroxyethoxy)-3-methylamino-4-nitrobenzol, 2,3-(Dihydroxypropoxy)-3-methylamino-4-nitrobenzol, 3-[(2-Aminoethyl)amino]-1-methoxy-4-nitrobenzol-hydrochlorid (HC Yellow 9), 1-Chlor-2,4-bis[(2-hydroxyethyl)amino]-5-nitrobenzol (HC Yellow 10), 2-[(2-Hydroxyethyl)amino]-5-nitrophenol (HC Yellow 11), 1-[(2'-Ureidoethyl)amino]-4-nitrobenzol, 1-Amino-4-[(2-aminoethyl)amino]-5-methyl-2-nitrobenzol, 4-[(2-Hydroxyethyl)amino]-3-nitro-1-methylbenzol, 1-Chlor-4-[(2-hydroxyethyl)amino]-3-nitrobenzol (HC Yellow 12), 4-[(2-Hydroxyethyl)amino]-3-nitro-1-trifluormethyl-benzol (HC Yellow 13).

Geeignete Chinonfarbstoffe sind insbesondere 1-[(2-Hydroxyethyl)amino]-4-methylamino-9,10-anthrachinon (C.I. 61,505, Disperse Blue 3), Mischungen aus 1,4-bis[(2-hydroxyethyl)amino]anthra-9,10-quinon mit 1-[(2-hydroxyethyl)amino]-4-[(3-hydroxypropyl)amino]anthra-9,10-quinon und 1,4-bis[(3-hydroxypropyl)amino]anthra-9,10-quinone (Disperse Blue 377), 1,4-Diamino-9,10-anthrachinon (C.I. 61,100, Disperse Violet 1), 1-Amino-4-(methylamino)-9,10-anthrachinon (C.I. 61,105, Disperse Violet 4, Solvent Violet No. 12), 2-Hydroxy-1,4-naphthochinon (Lawsone, C.I. 75,480, Natural Orange 6), 1,4-bis[(2,3-dihydroxypropyl)amino]-9,10-anthracenedion (HC Blue 14).

Geeignete neutrale Azofarbstoffe sind insbesondere 1-[Di(2-hydroxyethyl)amino]-3-methyl-4-[(4-nitrophenyl)azo]-benzol (C.I. 11,210, Disperse Red 17), 1-[Di(2-hydroxyethyl)amino]-4-[(4-nitrophenyl)azo]-benzol (Disperse Black 9), 4-[(4- Aminophenyl)azo]-1-[di(2-hydroxyethyl)amino]-3-methylbenzol (HC Yellow 7), 2,6-Diamino-3-[(pyridin-3-yl)azo]-pyridin, 4-[(4-Nitrophenyl)azo]-anilin (C.I. 11,005; Disperse Orange 3).

Bevorzugte nichtionische direktziehende Farbstoffe sind die unter den internationalen Bezeichnungen bzw. Handelsnamen HC Yellow 2, HC Yellow 4, HC Yellow 5, HC Yellow 6, HC Yellow 12, HC Orange 1, Disperse Orange 3, HC Red 1, HC Red 3, HC Red 10, HC Red 11, HC Red 13, HC Red BN, HC Blue 2, HC Blue 11, HC Blue 12, Disperse Blue 3, HC Violet 1, Disperse Violet 1, Disperse Violet 4, Disperse Black 9 bekannten Verbindungen, sowie 1,4-Diamino-2-nitrobenzol, 2-Amino-4-nitrophenol, 1,4-Bis-(2-hydroxyethyl)-amino-2-nitrobenzol, 3-Nitro-4-(2-hydroxyethyl)-aminophenol, 2-(2-Hydroxyethyl)amino-4,6-dinitrophenol, 4-[(2-Hydroxyethyl)amino]-3-nitro-1-methylbenzol, 1-Amino-4-(2-hydroxyethyl)-amino-5-chlor-2-nitrobenzol, 4-Amino-3-nitrophenol, 1-(2'-Ureidoethyl)amino-4-nitrobenzol, 2-[(4-Amino-2-nitrophenyl)amino]-benzoesäure, 6-Nitro-1,2,3,4-tetrahydrochinoxalin, 2-Hydroxy-1,4-naphthochinon, Pikraminsäure und deren Salze, 2-Amino-6-chloro-4-nitrophenol, 4-Ethylamino-3-nitrobenzoesäure und 2-Chlor-6-ethylamino-4-nitrophenol.

Es ist nicht erforderlich, dass die direktziehenden Farbstoffe jeweils einheitliche Verbindungen darstellen. Vielmehr können, bedingt durch die Herstellungsverfahren für die einzelnen Farbstoffe, in untergeordneten Mengen noch weitere Komponenten enthalten sein, soweit diese nicht das Färbeergebnis nachteilig beeinflussen oder aus anderen Gründen, z.B. toxikologischen, ausgeschlossen werden müssen.

Weiterhin können als direktziehende Farbstoffe auch in der Natur vorkommende Farbstoffe eingesetzt werden, wie sie beispielsweise in Henna rot, Henna neutral, Henna schwarz, Kamillenblüte, Sandelholz, schwarzem Tee, Faulbaumrinde, Salbei, Blauholz, Krappwurzel, Catechu, Sedre und Alkannawurzel enthalten sind.

Weiterhin hat es sich als vorteilhaft erwiesen, wenn die erfindungsgemäßen Mittel zur weiteren Nuancierung zusätzlich eine oder mehrere Farbstoffvorstufen naturanaloger Farbstoffe enthalten. Als Farbstoffvorstufen naturanaloger Farbstoffe werden bevorzugt solche Indole und Indoline eingesetzt, die mindestens zwei Gruppen ausgewählt aus Hydroxy- und/oder oder Aminogruppen, bevorzugt als Substituent am Sechsring, aufweisen. Diese Gruppen können weitere Substituenten tragen, z. B. in Form einer Veretherung oder Veresterung der Hydroxygruppe oder eine Alkylierung der Aminogruppe. In einer weiteren Ausführungsform enthalten die Färbemittel mindestens ein Indol- und/oder Indolinderivat. Erfindungsgemäße Zusammensetzungen, die Vorstufen naturanaloger Farbstoffe enthalten, werden bevorzugt als luftoxidative Färbemittel verwendet. In dieser Ausführungsform werden die besagten Zusammensetzungen folglich nicht mit einem zusätzlichen Oxidationsmittel versetzt.

Besonders bevorzugte Derivate des Indolins sind das 5,6-Dihydroxyindolin und das 2,3-Dioxoindolin (Isatin) und deren physiologisch verträglichen Salze.
Ein besonders bevorzugtes Derivat des Indols ist das 5,6-Dihydroxyindol und dessen physiologisch verträglichen Salze.

Als zweite wesentliche Komponente enthält das erfindungsgemäße Kit mindestens ein Mittel B, welches tensidfrei ist (Definition, s.o.) und mindestens ein Oxidationsmittel enthält. Als Oxidationsmittel kommen vorzugsweise Persulfate, Chlorite und insbesondere Wasserstoffperoxid oder dessen Anlagerungsprodukte an Harnstoff, Melamin sowie Natriumborat in Frage.

Ganz besonders bevorzugt sind erfindungsgemäße Wasserstoffperoxid oder dessen Anlagerungsprodukte an Harnstoff, Melamin sowie Natriumborat und Natriumcarbonat (gelegentlich auch als "Harnstoffperoxid", "Melaminperoxid" und "Natriumperborat" bzw. "Natriumpercarbonat" bezeichnet). Erfindungsgemäße bevrozugte Haarbehandlungs-Kits sind dadurch gekennzeichnet, daß das zweite Mittel (B), bezogen auf das Gewicht des Mittels B, 0,5 bis 20 Gew.-%, vorzugsweise 1,0 bis 17,5 Gew.-%, weiter bevorzugt 2,5 bis 16 Gew.-% und insbesondere 5 bis 14 Gew.-% Wasserstoffperoxid (berechnet als 100 %iges H₂O₂) enthält.

Erfindungsgemäß kann aber das Oxidationsfärbemittel auch zusammen mit einem Katalysator auf das Haar aufgebracht werden, der die Oxidation der Farbstoffvorprodukte, z.B. durch Luftsauerstoff, aktiviert. Solche Katalysatoren sind z.B. Metallionen, Iodide, Chinone oder bestimmte Enzyme.

Geeignete Metallionen sind beispielsweise Zn²+, Cu²⁺, Fe²⁺ , Fe³⁺, Mn²⁺, Mn⁴⁺, Li⁺, Mg²⁺, Ca²⁺ und Al³⁺. Besonders geeignet sind dabei Zn²⁺, Cu²⁺ und Mn²⁺. Die Metallionen können prinzipiell in der Form eines beliebigen, physiologisch verträglichen Salzes oder in Form einer Komplexverbindung eingesetzt werden. Bevorzugte Salze sind die Acetate, Sulfate, Halogenide, Lactate und Tartrate. Durch Verwendung dieser Metallsalze kann sowohl die Ausbildung der Färbung beschleunigt als auch die Farbnuance gezielt beeinflusst werden.

Geeignete Enzyme sind z.B. Peroxidasen, die die Wirkung geringer Mengen an Wasserstoffperoxid deutlich verstärken können. Weiterhin sind solche Enzyme erfindungsgemäß geeignet, die mit Hilfe von Luftsauerstoff die Oxidationsfarbstoffvorprodukte direkt oxidieren, wie beispielsweise die Laccasen, oder *in situ* geringe Mengen Wasserstoffperoxid erzeugen und auf diese Weise die Oxidation der Farbstoffvorprodukte biokatalytisch aktivieren. Besonders geeignete Katalysatoren für die Oxidation der Farbstoffvorläufer sind die sogenannten 2-Elektronen-Oxidoreduktasen in Kombination mit den dafür spezifischen Substraten, z.B.
- Pyranose-Oxidase und z.B. D-Glucose oder Galactose,
- Glucose-Oxidase und D-Glucose,
- Glycerin-Oxidase und Glycerin,
- Pyruvat-Oxidase und Benztraubensäure oder deren Salze,
- Alkohol-Oxidase und Alkohol (MeOH, EtOH),
- Lactat-Oxidase und Milchsäure und deren Salze,
- Tyrosinase-Oxidase und Tyrosin,
- Uricase und Harnsäure oder deren Salze,
- Cholinoxidase und Cholin,
- Aminosäure-Oxidase und Aminosäuren.

Als dritte wesentliche Komponente enthält das erfindungsgemäße Kit mindestens ein Mittel C, welches Tensid(e) enthält. Als oberflächenaktive Stoffe können nichtionische, anionische, kationische und/oder amphotere bzw. amphotere Tenside enthalten sein, deren Anteil an den Mitteln C üblicherweise bei etwa 5 bis 99 und vorzugsweise 7 bis 90 Gew.-% beträgt. Typische Beispiele für anionische Tenside sind Seifen, Alkylbenzolsulfonate, Alkansulfonate, Olefinsulfonate, Alkylethersulfonate, Glycerinethersulfonate, α-Methylestersulfonate, Sulfofettsäuren, Alkylsulfate, Fettalkoholethersulfate, Glycerinethersulfate, Hydroxymischethersulfate, Monoglycerid-(ether)sulfate, Fettsäureamid(ether)sulfate, Mono- und Dialkyl-sulfosuccinate, Mono- und Dialkylsulfosuccinamate, Sulfotriglyceride, Amidseifen, Ethercarbonsäuren und deren Salze, Fettsäureisethionate, Fettsäuresarcosinate, Fettsäuretauride, N-Acylaminosäuren, wie beispielsweise Acyllactylate, Acyltartrate, Acylglutamate und Acylaspartate, Alkyloligoglucosidsulfate, Proteinfettsäurekondensate (insbesondere pflanzliche Produkte auf Weizenbasis) und Alkyl(ether)phosphate. Sofern die anionischen Tenside Polyglycoletherketten enthalten, können diese eine konventionelle, vorzugsweise jedoch eine eingeengte Homologenverteilung aufweisen. Typische Beispiele für nichtionische Tenside sind Fettalkoholpolyglycolether, Alkylphenolpolyglycolether, Fettsäurepolyglycolester, Fettsäureamidpolyglycolether, Fettaminpolyglycolether, alkoxylierte Triglyceride, Mischether bzw. Mischformale, gegebenenfalls partiell oxidierte Alk(en)yloligoglykoside bzw. Glucoronsäurederivate, Fettsäure-N-alkylglucamide, Proteinhydrolysate (insbesondere pflanzliche Produkte auf Weizenbasis), Polyolfettsäureester, Zuckerester, Sorbitanester, Polysorbate und Aminoxide. Sofern die nicht-ionischen Tenside Polyglycoletherketten enthalten, können diese eine konventionelle, vorzugsweise jedoch eine eingeengte Homologenverteilung aufweisen. Typische Beispiele für kationische Tenside sind quartäre Ammoniumverbindungen und Esterquats, insbesondere quaternierte Fettsäuretrialkanolaminestersalze. Typische Beispiele für amphotere bzw. zwitterionische Tenside sind Alkylbetaine, Alkylamidobetaine, Aminopropionate, Aminoglycinate, Imidazoliniumbetaine und Sulfobetaine. Bei den genannten Tensiden handelt es sich ausschließlich um bekannte Verbindungen. Hinsichtlich Struktur und Herstellung dieser Stoffe sei auf einschlägige Übersichtsarbeiten beispielsweise J.Falbe (ed.), "Surfactants in Consumer Products", Springer Verlag, Berlin, 1987, S. 54-124 oder J.Falbe (ed.), "Katalysatoren, Tenside und Mineralöladditive", Thieme Verlag, Stuttgart, 1978, S. 123-217 verwiesen.

Besonders bevorzugte erfindungsgemäße Haarbehandlungs-Kits sind dadurch gekennzeichnet, daß das dritte Mittel (C), bezogen auf das Gewicht des Mittels C, 2,5 bis 100 Gew.-%, vorzugsweise 5 bis 95 Gew.-%, weiter bevorzugt 7,5 bis 90 Gew.-% und insbesondere 10 bis 75 Gew.-% Tensid(e) enthält.

Es hat sich gezeigt, daß eine Kombination aus mindestens einem oder mehreren Zuckertensiden und mindestens einem oder mehreren amphoteren Tensiden besonders vorteilhafte Schäume erzielt, die stabil sind und eine hervorragend gleichmäßige Färbung ermöglichen. Insbesondere, wenn das Mengenverhältnis von Zuckertensid(en) zu amphoteren Tensid(en) mindestens 2:1 beträgt, werden erfindungsgemäß besonders gute Ergebnisse erzielt.

Gemäß einer ersten bevorzugten Ausführungsform enthält das Mittel als Zuckertensid mindestens ein Alkyl- oder Alkenyloligoglykosid. Diese Zuckertenside stellen bekannte nichtionische Tenside gemäß Formel dar,

R¹O-[G]ₚ

in der R¹ für einen Alkyl- oder Alkenylrest mit 4 bis 22 Kohlenstoffatomen, G für einen Zuckerrest mit 5 oder 6 Kohlenstoffatomen und p für Zahlen von 1 bis 10 steht.

Die Alkyl- und Alkenyloligoglykoside können sich von Aldosen beziehungsweise Ketosen mit 5 oder 6 Kohlenstoffatomen, vorzugsweise von Glucose, ableiten. Die bevorzugten Alkyl- und/oder Alkenyloligoglykoside sind somit Alkyl- und/oder Alkenyloligoglucoside. Die Indexzahl p in der allgemeinen Formel (II) gibt den Oligomerisierungsgrad (DP), d. h. die Verteilung von Mono- und Oligoglykosiden an und steht für eine Zahl zwischen 1 und 10. Während p im einzelnen Molekül stets ganzzahlig sein muß und hier vor allem die Werte p = 1 bis 6 annehmen kann, ist der Wert p für ein bestimmtes Alkyloligoglykosid eine analytisch ermittelte rechnerische Größe, die meistens eine gebrochene Zahl ist. Vorzugsweise werden Alkyl- und/oder Alkenyloligoglykoside mit einem mittleren Oligomerisierungsgrad p von 1,1 bis 3,0 eingesetzt. Aus anwendungstechnischer Sicht sind solche Alkyl- und/oder Alkenyloligoglykoside bevorzugt, deren Oligomerisierungsgrad kleiner als 1,7 ist und insbesondere zwischen 1,2 und 1,4 liegt.

Der Alkyl- bzw. Alkenylrest R¹ kann sich von primären Alkoholen mit 4 bis 11, vorzugsweise 8 bis 10 Kohlenstoffatomen ableiten. Typische Beispiele sind Butanol, Capronalkohol, Caprylalkohol, Caprinalkohol und Undecylalkohol sowie deren technische Mischungen, wie sie beispielsweise bei der Hydrierung von technischen Fettsäuremethylestern oder im Verlauf der Hydrierung von Aldehyden aus der Roelen'schen Oxosynthese erhalten werden. Bevorzugt sind Alkyloligoglucoside der Kettenlänge C₈-C₁₀ (DP = 1 bis 3), die als Vorlauf bei der destillativen Auftrennung von technischem C₈-C₁₈-Kokosfettalkohol anfallen und mit einem Anteil von weniger als 6 Gew.-% C₁₂-Alkohol verunreinigt sein können sowie Alkyloligoglucoside auf Basis technischer C_{9/11}-Oxoalkohole (DP = 1 bis 3). Der Alkyl- bzw. Alkenylrest R¹ kann sich ferner auch von primären Alkoholen mit 12 bis 22, vorzugsweise 12 bis 14 Kohlenstoffatomen ableiten. Typische Beispiele sind Laurylalkohol, Myristylalkohol, Cetylalkohol, Palmoleylalkohol, Stearylalkohol, Isostearylalkohol, Oleylalkohol, Elaidylalkohol, Petroselinylalkohol, Arachylalkohol, Gadoleylalkohol, Behenylalkohol, Erucylalkohol, Brassidylalkohol sowie deren technische Gemische, die wie oben beschrieben erhalten werden können. Bevorzugt sind Alkyloligoglucoside auf Basis von gehärtetem C_{12/14}-Kokosalkohol mit einem DP von 1 bis 3.

Gemäß einer zweiten Ausführungsform (C2) der Erfindung handelt es sich bei dem Zuckertensid um ein Fettsäure-N-alkylpolyhydroxyalkylamid, ein nichtionisches Tensid der Formel (III), 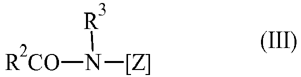 in der R²CO für einen aliphatischen Acylrest mit 6 bis 22 Kohlenstoffatomen, R³ für Wasserstoff, einen Alkyl- oder Hydroxyalkylrest mit 1 bis 4 Kohlenstoffatomen und [Z] für einen linearen oder verzweigten Polyhydroxyalkylrest mit 3 bis 12 Kohlenstoffatomen und 3 bis 10 Hydroxylgruppen steht.

Vorzugsweise leiten sich die Fettsäure-N-alkylpolyhydroxyalkylamide von reduzierenden Zuckern mit 5 oder 6 Kohlenstoffatomen, insbesondere von der Glucose, ab. Die bevorzugten Fettsäure-N-alkylpolyhydroxyalkylamide stellen daher Fettsäure-N-alkylglucamide dar, wie sie durch die Formel (IV) wiedergegeben werden:

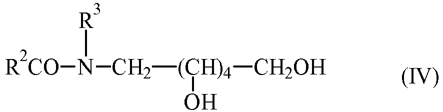

Vorzugsweise werden als Fettsäure-N-alkylpolyhydroxyalkylamide Glucamide der Formel (IV) eingesetzt, in der R³ für Wasserstoff oder eine Alkylgruppe steht und R²CO für den Acylrest der Capronsäure, Caprylsäure, Caprinsäure, Laurinsäure, Myristinsäure, Palmitinsäure, Palmoleinsäure, Stearinsäure, Isostearinsäure, Ölsäure, Elaidinsäure, Petroselinsäure, Linolsäure, Linolensäure, Arachinsäure, Gadoleinsäure, Behensäure oder Erucasäure bzw. derer technischer Mischungen steht. Besonders bevorzugt sind Fettsäure-N-alkylglucamide der Formel (IV), die durch reduktive Aminierung von Glucose mit Methylamin und anschließende Acylierung mit Laurinsäure oder C_{12/14}-Kokosfettsäure bzw. einem entsprechenden Derivat erhalten werden. Weiterhin können sich die Polyhydroxyalkylamide auch von Maltose und Palatinose ableiten.

Das Zuckertensid ist in den erfindungsgemäßen Mitteln bevorzugt in Mengen von 0,1 - 20 Gew.-%, bezogen auf das anwendungsbereite Mittel, enthalten. Mengen von 4 - 15 Gew.-% sind besonders bevorzugt.

Innerhalb der Gruppe der amphoteren Tenside sind sowohl die zwitterionischen als auch die ampholytischen Tenside erfindungsgemäß vorteilhaft.

Als zwitterionische Tenside werden solche oberflächenaktiven Verbindungen bezeichnet, die im Molekül mindestens eine quartäre Ammoniumgruppe und mindestens eine -COO⁽⁻⁾ - oder -SO₃⁽⁻⁾ - Gruppe tragen, wobei die Verbindungen, die eine -COO⁽⁻⁾ tragen, erfindungsgemäß ganz besonders bevorzugt sind. Besonders geeignete zwitterionische Tenside sind die so genannten Betaine wie die N-Alkyl-N,N-dimethylammonium-glycinate, beispielsweise das Kokosalkyl-dimethylammoniumglycinat, N-Acyl-aminopropyl-N,N-dimethylammoniumglycinate, beispielsweise das Kokosacylaminopropyl-dimethylammoniumglycinat, und 2-Alkyl-3-carboxymethyl-3-hydroxyethyl-imidazoline mit jeweils 8 bis 18 C-Atomen in der Alkyl- oder Acylgruppe sowie das Kokosacylaminoethylhydroxyethylcarboxymethylglycinat. Ein bevorzugtes zwitterionisches Tensid ist das unter der INCI-Bezeichnung Cocamidopropyl Betaine bekannte Fettsäureamid-Derivat.

Unter ampholytischen Tensiden werden solche oberflächenaktiven Verbindungen verstanden, die außer einer C₈ - C₂₄- Alkyl- oder -Acylgruppe im Molekül mindestens eine freie Aminogruppe und mindestens eine -COOH- oder -SO₃H-Gruppe enthalten und zur Ausbildung innerer Salze befähigt sind. Beispiele für geeignete ampholytische Tenside sind N-Alkylglycine, N-Alkylpropionsäuren, N-Alkylaminobuttersäuren, N-Alkyliminodipropionsäuren, N-Hydroxyethyl-N-alkylamidopropylglycine, N-Alkyltaurine, N-Alkylsarcosine, 2-Alkylaminopropionsäuren und Alkylaminoessigsäuren mit jeweils etwa 8 bis 24 C-Atomen in der Alkylgruppe. Besonders bevorzugte ampholytische Tenside sind das N-Kokosalkylaminopropionat, das Kokosacylaminoethylaminopropionat und das C₁₂ - C₁₈-Acylsarcosin.

Die amphoteren Tenside sind in dem Mittel vorzugsweise in Mengen von 0,1 bis 10 Gew.-%, vorzugsweise von 1 bis 5 Gew.-%, jeweils bezogen auf das anwendungsbereite Mittel, d.h. die Mischung der Mittel A, B und C, enthalten.

Weiterhin hat es sich als erfindungsgemäß als vorteilhaft zur Erzielung der gewünschten Schaumeigenschaften erwiesen, wenn die anwendungsbereiten Mittel einen deutlichen Überschuss an Zuckertensid(en) gegenüber dem oder den amphoteren Tensiden enthalten. Ein Mengenverhältnis von Zuckertensid(en) zu amphoter(en) Tensiden von mindestens 2:1 ist erfindungsgemäß bevorzugt, weiter bevorzugt ist ein Mengenverhältnis von mindestens 3:1.
Eine weitere, überraschende Verbesserung der Pflegeleistung der Färbemittel ohne Verringerung der Schaumqualität konnte erfindungsgemäß erzielt werden durch die Zugabe mindestens eines kationischen Tensides. Erfindungsgemäß bevorzugt sind insbesondere kationische Tenside vom Typ der quartären Ammoniumverbindungen und der Esterquats.
Bevorzugte quaternäre Ammoniumverbindungen sind ausgewählt aus den Verbindungen der Formel (I) 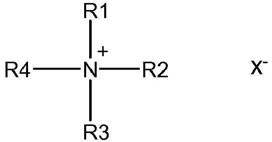 wobei die Reste R1, R2, R3 und R4 stehen unabhängig voneinander für eine C₁-C₂₆-Alkylgruppe, mit der Maßgabe, dass mindestens einer dieser Reste steht für eine C₁₀-C₂₆-Alkylgruppe und X⁻ steht für ein einwertiges Anion.

Dabei hat es sich als erfindungsgemäß bevorzugt erwiesen, wenn einer, zwei oder drei der Reste R1, R2, R3 und R4 für eine C₁-C₄-Alkylgruppe, insbesondere eine Methylgruppe, stehen, und die übrigen Reste unabhängig voneinander für eine C₁₂-C₂₂-Alkylgruppe stehen. Als Anion sind insbesondere die Halogenidanionen, insbesondere das Chloridanion sowie das Bromidanion, erfindungsgemäß bevorzugt.

Besonders bevorzugt sind C₁₀-C₂₆-Alkyltrimethylammoniumchloride, C₁₀-C₂₆-Dialkyldimethyl-ammoniumchloride und C₁₀-C₂₆-Trialkylmethylammoniumchloride, beispielsweise Cetyltrimethylammoniumchlorid, Stearyltrimethylammoniumchlorid, Distearyldimethylammoniumchlorid, Lauryldimethylammoniumchlorid, Lauryldimethylbenzylammoniumchlorid und Tricetylmethylammoniumchlorid.

Bei den Esterquats handelt es sich um bekannte Stoffe, die sowohl mindestens eine Esterfunktion als auch mindestens eine quartäre Ammoniumgruppe als Strukturelement enthalten. Bevorzugte Esterquats sind quaternierte Estersalze von Fettsäuren mit Triethanolamin, quaternierte Estersalze von Fettsäuren mit Diethanolalkylaminen und quaternierten Estersalzen von Fettsäuren mit 1,2-Dihydroxypropyldialkylaminen. Solche Produkte werden beispielsweise unter den Warenzeichen Stepantex®, Dehyquart® und Armocare® vertrieben. Die Produkte Armocare® VGH-70, ein N,N-Bis(2-Palmitoyloxyethyl)dimethylammoniumchlorid, sowie Dehyquart® F-75, Dehyquart® C-4046, Dehyquart® L80 und Dehyquart® AU-35 sind Beispiele für solche Esterquats.

Ganz besonders bevorzugt im Rahmen der vorliegenden Erfindung ist es, wenn die Mittel als kationisches Tensid zumindest ein Stearyltrimehtylammoniumsalz oder ein Cetyltrimethylammoniumsalz enthalten.

Die erfindungsgemäßen Mittel enthalten das oder die kationischen Tenside vorzugsweise in einer Menge von 0,05 bis 5 Gew.-%, insbesondere von 0,1 bis 1 Gew.-% jeweils bezogen auf das anwendungsbereite Mittel, d.h. die Mischung der Mittel A, B und C.

Weiterhin hat es sich als vorteilhaft erwiesen, wenn die erfindungsgemäßen Mittel mindestens ein weiteres nichtionisches Tensid enthalten, das von den Zuckertensiden verschieden ist.

Dabei hat es sich als besonders vorteilhaft erwiesen, wenn das weitere nichtionische Tensid einen HLB-Wert oberhalb von 10, vorzugsweise oberhalb von 14 aufweist. In dieser Gruppe wiederum sind die nichtionischen Tenside besonders bevorzugt, die Ethylenoxid- und/oder Propylenoxidgruppen enthalten. Ganz besonders bevorzugt sind nichtionische Tenside mit mehr als 29 Ethylenoxideinheiten. Neben den entsprechend ethoxylierten Fettalkoholen sind erfindungsgemäß insbesondere die Anlagerungsprodukte von 30 bis 60 Mol Ethylenoxid an Rizinusöl und gehärtetes Rizinusöl besonders geeignet. Diese speziellen nichtionischen Tenside ermöglichen eine weitere Verbesserung der Schaumkonsistenz, insbesondere im Hinblick auf eine höhere Festigkeit, eine gesteigerte Feinporigkeit und eine größere Geschmeidigkeit.

Ferner konnte in den dieser Erfindung zugrunde liegenden Arbeiten gezeigt werden, dass es vorteilhaft ist, wenn die Mittel neben den oben genannten Tensiden mindestens ein anionisches Tensid aufweist. Als anionische Tenside sind
- lineare und verzweigte Fettsäuren mit 8 bis 30 C-Atomen (Seifen),
- Ethercarbonsäuren der Formel R-O-(CH₂CH₂O)ₓ-CH₂COOH, in der R eine lineare Alkylgruppe mit 8 bis 30 C-Atomen und x = 0 oder 1 bis 16 ist, und
- Alkylsulfate und Alkylpolyglykolethersulfate der Formel R-O(CH₂-CH₂O)ₓ-OSO₃H, in der R eine bevorzugt lineare Alkylgruppe mit 8 bis 30 C-Atomen und x = 0 oder 1 bis 12 ist,
erfindungsgemäß besonders bevorzugt.

In einer ganz besonders bevorzugten Ausführungsform der vorliegenden Erfindung enthalten die Mittel neben den erfindungswesentlichen Tensiden weiterhin ein nichtionisches Tensid, das von den Zuckertensiden verschieden ist, ein kationisches Tensid sowie ein anionisches Tensid. Hinsichtlich der bevorzugten Vertreter der zusätzlichen Tenside in dieser Kombination sei an dieser Stelle auf die obigen Ausführungen verwiesen.

Zusammenfassend sind erfindungsgemäße Haarbehandlungs-Kits bevorzuugt, die dadurch gekennzeichnet sind, daß die Mischung der Mittel A, B und C bezogen auf das Gewicht der Mischung mindestens 10,5 Gew.-%, vorzugsweise mindestens 11 Gew.-%, weiter bevorzugt mindestens 12 Gew.-%, noch weiter bevorzugt mindestens 13 Gew.-% und insbesondere mindestens 14 Gew.-% Tensid(e) enthält.

Überraschenderweise konnte gezeigt werden, dass die Anwesenheit geringer Mengen eines polymeren Verdickers die Schaumbeständigkeit positiv beeinflussen konnte. Daher ist es im Rahmen einer Ausführungsform der vorliegenden Erfindung bevorzugt, wenn die Mittel mindestens einen polymeren Verdicker enthalten.

Beispiele für erfindungsgemäß bevorzugte polymere Verdicker sind:
Acrylamides Copolymer, Acrylamide/Sodium Acrylate Copolymer, Acrylamide/Sodium Acryloyldimethyltaurate Copolymer, Acrylates/Acetoacetoxyethyl Methacrylate Copolymer, Acrylates/Beheneth-25 Methacrylate Copolymer, Acrylates/C10-30 Alkyl Acrylate Crosspolymer, Acrylates/Ceteth-20 Itaconate Copolymer, Acrylates/Ceteth-20 Methacrylate Copolymer, Acrylates/Laureth-25 Methacrylate Copolymer, Acrylates/Palmeth-25 Acrylate Copolymer, Acrylates/Palmeth-25 Itaconate Copolymer, Acrylates/Steareth-50 Acrylate Copolymer, Acrylates/Steareth-20 Itaconate Copolymer, Acrylates/Steareth-20 Methacrylate Copolymer, Acrylates/Stearyl Methacrylate Copolymer, Acrylates/Vinyl Isodecanoate Crosspolymer, Acrylic Acid/Acrylonitrogens Copolymer, Agar, Agarose, Alcaligenes Polysaccharides, Algin, Alginic Acid, Ammonium Acrylates/Acrylonitrogens Copolymer, Ammonium Acrylates Copolymer, Ammonium Acryloyldimethyltaurate/Vinyl Formamide Copolymer, Ammonium Acryloyldimethyltaurate/VP Copolymer, Ammonium Alginate, Ammonium Polyacryloyldimethyl Taurate, Amylopectin, Ascorbyl Methylsilanol Pectinate, Astragalus Gummifer Gum, Attapulgite, Avena Sativa (Oat) Kernel Flour, Bentonite, Butoxy Chitosan, Caesalpinia Spinosa Gum, Calcium Alginate, Calcium Carboxymethyl Cellulose, Calcium Carrageenan, Calcium Potassium Carbomer, Calcium Starch Octenylsuccinate, C20-40 Alkyl Stearate, Carbomer, Carboxybutyl Chitosan, Carboxymethyl Chitin, Carboxymethyl Chitosan, Carboxymethyl Dextran, Carboxymethyl Hydroxyethylcellulose, Carboxymethyl Hydroxypropyl Guar, Cellulose Acetate Propionate Carboxylate, Cellulose Gum, Ceratonia Siliqua Gum, Cetyl Hydroxyethylcellulose, Cholesterol/HDI/Pullulan Copolymer, Cholesteryl Hexyl Dicarbamate Pullulan, Cyamopsis Tetragonoloba (Guar) Gum, Diglycol/CHDM/Isophthalates/SIP Copolymer, Dihydrogenated Tallow Benzylmonium Hectorite, Dimethicone Crosspolymer-2, Dimethicone Propyl PG-Betaine, DMAPA Acrylates/Acrylic Acid/Acrylonitrogens Copolymer, Ethylene/Sodium Acrylate Copolymer, Gelatin, Gellan Gum, Glyceryl Alginate, Glycine Soja (Soybean) Flour, Guar Hydroxypropyltrimonium Chloride, Hectorite, Hydrated Silica, Hydrogenated Potato Starch, Hydroxybutyl Methylcellulose, Hydroxyethyl Acrylate/Sodium Acryloyldimethyl Taurate Copolymer, Hydroxyethylcellulose, Hydroxyethyl Chitosan, Hydroxyethyl Ethylcellulose, Hydroxypropylcellulose, Hydroxypropyl Chitosan, Hydroxypropyl Ethylenediamine Carbomer, Hydroxypropyl Guar, Hydroxypropyl Methylcellulose, Hydroxypropyl Methylcellulose Stearoxy Ether, Hydroxypropyl Starch, Hydroxypropyl Starch Phosphate, Hydroxypropyl Xanthan Gum, Hydroxystearamide MEA, Isobutylene/Sodium Maleate Copolymer, Lithium Magnesium Silicate, Lithium Magnesium Sodium Silicate, Macrocystis Pyrifera (Kelp), Magnesium Alginate, Magnesium Aluminum Silicate, Magnesium Silicate, Magnesium Trisilicate, Methoxy PEG-22/Dodecyl Glycol Copolymer, Methylcellulose, Methyl Ethylcellulose, Methyl Hydroxyethylcellulose, Microcrystalline Cellulose, Montmorillonite, Moroccan Lava Clay, Natto Gum, Nonoxynyl Hydroxyethylcellulose, Octadecene/MA Copolymer, Pectin, PEG-800, PEG-Crosspolymer, PEG-150/Decyl Alcohol/SMDI Copolymer, PEG-175 Diisostearate, PEG-190 Distearate, PEG-15 Glyceryl Tristearate, PEG-140 Glyceryl Tristearate, PEG-240/HDI Copolymer Bis-Decyltetradeceth-20 Ether, PEG-100/IPDI Copolymer, PEG-180/Laureth-50/TMMG Copolymer, PEG-10/Lauryl Dimethicone Crosspolymer, PEG-15/Lauryl Dimethicone Crosspolymer, PEG-2M, PEG-5M, PEG-7M, PEG-9M, PEG-14M, PEG-20M, PEG-23M, PEG-25M, PEG-45M, PEG-65M, PEG-90M, PEG-115M, PEG-160M, PEG-120 Methyl Glucose Trioleate, PEG-180/Octoxynol-40/TMMG Copolymer, PEG-150 Pentaerythrityl Tetrastearate, PEG-4 Rapeseedamide, PEG-150/Stearyl Alcohol/SMDI Copolymer, Polyacrylate-3, Polyacrylic Acid, Polycyclopentadiene, Polyether-1, Polyethylene/Isopropyl Maleate/MA Copolyol, Polymethacrylic Acid, Polyquaternium-52, Polyvinyl Alcohol, Potassium Alginate, Potassium Aluminum Polyacrylate, Potassium Carbomer, Potassium Carrageenan, Potassium Polyacrylate, Potato Starch Modified, PPG-14 Laureth-60 Hexyl Dicarbamate, PPG-14 Laureth-60 Isophoryl Dicarbamate, PPG-14 Palmeth-60 Hexyl Dicarbamate, Propylene Glycol Alginate, PVP/Decene Copolymer, PVP Montmorillonite, Rhizobian Gum, Ricinoleic Acid/Adipic Acid/AEEA Copolymer, Sclerotium Gum, Sodium Acrylate/Acryloyldimethyl Taurate Copolymer, Sodium Acrylates/Acrolein Copolymer, Sodium Acrylates/Acrylonitrogens Copolymer, Sodium Acrylates Copolymer, Sodium Acrylates/Vinyl Isodecanoate Crosspolymer, Sodium Acrylate/Vinyl Alcohol Copolymer, Sodium Carbomer, Sodium Carboxymethyl Chitin, Sodium Carboxymethyl Dextran, Sodium Carboxymethyl Beta-Glucan, Sodium Carboxymethyl Starch, Sodium Carrageenan, Sodium Cellulose Sulfate, Sodium Cyclodextrin Sulfate, Sodium Hydroxypropyl Starch Phosphate, Sodium Isooctylene/MA Copolymer, Sodium Magnesium Fluorosilicate, Sodium Polyacrylate, Sodium Polyacrylate Starch, Sodium Polyacryloyldimethyl Taurate, Sodium Polymethacrylate, Sodium Polystyrene Sulfonate, Sodium Silicoaluminate, Sodium Starch Octenylsuccinate, Sodium Stearoxy PG-Hydroxyethylcellulose Sulfonate, Sodium Styrene/Acrylates Copolymer, Sodium Tauride Acrylates/Acrylic Acid/Acrylonitrogens Copolymer, Solanum Tuberosum (Potato) Starch, Starch/Acrylates/Acrylamide Copolymer, Starch Hydroxypropyltrimonium Chloride, Steareth-60 Cetyl Ether, Steareth-100/PEG-136/HDI Copolymer, Sterculia Urens Gum, Synthetic Fluorphlogopite, Tamarindus Indica Seed Gum, Tapioca Starch, TEA-Alginate, TEA-Carbomer, Triticum Vulgare (Wheat) Starch, Tromethamine Acrylates/Acrylonitrogens Copolymer, Tromethamine Magnesium Aluminum Silicate, Welan Gum, Xanthan Gum, Yeast Beta-Glucan, Yeast Polysaccharides, Zea Mays (Corn) Starch.

Aus dieser umfangreichen Gruppe haben sich die Verdickungsmittel als besonders vorteilhaft erwiesen, die mindestens ein Monomer vom Typ der Acrylsäure oder Methacrylsäure sowie derer Derivate enthalten. Ein erfindungsgemäß ganz besonders bevorzugtes Polymer ist das unter der INCI-Bezeichnung Acrylates Copolymer bekannte Copolymer aus zwei oder mehr Monomeren, ausgewählt aus Acrylsäure, Methacrysäure und deren Ester mit C₁-C₄-Alkylgruppen.

Wie bereits ausgeführt, ist es besonders vorteilhaft, wenn der polymere Verdicker in geringen Mengen, vorzugsweise in Mengen von 0,05 bis 2 Gew.-%, insbesondere von 0,1 bis 1 Gew.-%, jeweils bezogen auf die anwendungsbereite Mischung, enthalten ist.

Eine weitere Verbesserung der pflegenden Eigenschaften des Produktes konnte durch den Einsatz eines Extraktes, der aus und/oder mithilfe von Algen und/oder Plankton gewonnen wird, erzielt werden. Insbesondere der Feuchtigkeitshaushalt der Fasern als auch deren Glanz konnte durch diese Extrakte erheblich gesteigert werden. Unter "Extrakten, die aus und/oder mithilfe von Algen und/oder Plankton gewonnen werden" sind erfindungsgemäß Wirkstoffmischungen zu verstehen, die entweder durch Extraktion von Algen und/oder Plankton selbst oder durch Extraktion der die Algen und/oder das Plankton umgebenen Wasserphase gewonnen werden.
Erfindungsgemäß bevorzugte Algen und/oder Planktonarten sind ausgewählt aus den Gattungen Haptophyta, Schlundgeißler (Cryptista), Euglenozoa, Dinozoa, Chlorarachniophyta, Goldalgen (Chrysophyta), Kieselalgen (Bacillariophyta, auch Diatomeen genannt), Braunalgen (Phaeophyta), Dinogellaten, Rotalgen (Rhodophyta), Grünalgen (Chlorophyta), Picobiliphyta sowie der Blaualgen (beispielsweise Oscillatoria und Spirulina)

Besonders bevorzugt sind im Rahmen der vorliegenden Erfindung Extrakte aus vornehmlich in Süßwasser vorkommenden Blaualgen.

Hinsichtlich der Art und Weise, wie die erfindungsgemäßen Extrakte aus den Algen- und/oder Planktonbestandteilen gewonnen wird, bestehen prinzipiell keine Einschränkungen.

Als Extraktionsmittel zur Herstellung der genannten Algenextrakte können Wasser, Alkohole sowie deren Mischungen verwendet werden. Unter den Alkoholen sind dabei niedere Alkohole wie Ethanol und Isopropanol, insbesondere aber mehrwertige Alkohole wie Ethylenglykol und Propylenglykol, sowohl als alleiniges Extraktionsmittel als auch in Mischung mit Wasser, bevorzugt. Blaualgenextrakte, die mittels einer Wasser/Propylenglykol-Mischung gewonnen werden, haben sich als besonders geeignet erwiesen. Es hat sich dabei als besonders geeignet erwiesen, wenn diese Extraktionsmittel in einem Verhältnis von 1:10 bis 10:1 eingesetzt werden.

Weiterhin kann es erfindungsgemäß bevorzugt sein, Extrakte einzusetzen, die vor der Anwendung zumindest teilweise entfärbt wurden. Dies kann beispielsweise durch Nutzung von Aktivkohle erfolgen.

Ebenso ist möglich, in den erfindungsgemäßen Mitteln als Algen- oder Plankton-Extrakt die wässrige Aufzucht- beziehungsweise Kulturlösung von Algen oder Plankton einzusetzen. Dazu werden die Algen oder das Plankton zunächst mit einer physikalischen Trennmethode, wie Filtration oder Zentrifugation von der Kulturlösung abgetrennt.

In einer besonders bevorzugten Ausführungsform der vorliegenden Erfindung enthält das Mittel einen Extrakt einer Blaualge, vorzugsweise einer Süßwasserblaualge, ganz besonders bevorzugt einer Blaualge der Gattung Spirulina.

Die erfindungsgemäßen Mittel enthalten die Algen- und/oder Planktonextrakte vorzugsweise in einer Menge von 0,001 bis 5 Gew.-%, vorzugsweise von 0,01 bis 2 Gew.-% bezogen auf das anwendungsbereite Mittel.

In einer weiteren bevorzugten Ausführungsform enthalten die erfindungsgemäßen Mittel mindestens einen Wirkstoff, der aus Pflanzen der Gattung Aloe Vera gewonnen wird und für diese Pflanzengattung charakteristisch ist. Die Zugabe dieser Komponente ermöglicht eine überraschen intensive Verbesserung des Feuchtigkeithaushaltes der Fasern.

Weiterhin kann es erfindungsgemäß bevorzugt sein, wenn die Mittel zusätzlich mindestens eine Aminosäure und/oder mindestens ein Protein. Erfindungsgemäß bevorzugte Aminosäuren sind Arginin, Serin, Lysin, Glycin, Tyrosin, Prolin, Glutamin, Cystein und Histidin. Der Aminosäuren und/oder die Proteine ermöglicht eine überraschend stark ausfallende Strukturgebung der Haare.

Im Rahmen einer sechsten bevorzugten Ausführungsform enthalten die erfindungsgemäßen Zubereitungen mindestens einen Pflanzenextrakt.

Üblicherweise werden diese Extrakte durch Extraktion der gesamten Pflanze hergestellt. Es kann aber in einzelnen Fällen auch bevorzugt sein, die Extrakte ausschließlich aus Blüten und/oder Blättern der Pflanze herzustellen.

Hinsichtlich der erfindungsgemäß bevorzugten Pflanzenextrakte wird insbesondere auf die Extrakte hingewiesen, die in der auf Seite 44 der 3. Auflage des Leitfadens zur Inhaltsstoffdeklaration kosmetischer Mittel, herausgegeben vom Industrieverband Körperpflege- und Waschmittel e.V. (IKW), Frankfurt, beginnenden Tabelle aufgeführt sind.

Erfindungsgemäß sind vor allem die Extrakte aus Moringa Olifeira, Grünem Tee, Eichenrinde, Brennnessel, Hamamelis, Hopfen, Henna, Kamille, Klettenwurzel, Schachtelhalm, Weißdorn, Lindenblüten, Mandel, Fichtennadel, Rosskastanie, Sandelholz, Wacholder, Kokosnuss, Mango, Aprikose, Limone, Weizen, Kiwi, Melone, Orange, Grapefruit, Salbei, Rosmarin, Birke, Malve, Wiesenschaumkraut, Quendel, Schafgarbe, Thymian, Melisse, Hauhechel, Huflattich, Eibisch, Meristem, Ginseng und Ingwerwurzel bevorzugt.

Besonders bevorzugt sind die Extrakte aus Moringa Olifeira, Grünem Tee, Eichenrinde, Brennnessel, Hamamelis, Hopfen, Kamille, Klettenwurzel, Schachtelhalm, Lindenblüten, Mandel, Kokosnuss, Mango, Aprikose, Limone, Weizen, Kiwi, Melone, Orange, Grapefruit, Salbei, Rosmarin, Birke, Wiesenschaumkraut, Quendel, Schafgarbe, Hauhechel, Meristem, Ginseng und Ingwerwurzel.

Auch Extrakte aus Blüten haben sich erfindungsgemäß als besonders bevorzugt erwiesen. So können die Mittel beispielsweise einen Extrakt aus Seerosen, Orchideen, Wildrosen, Lotusblüten oder Kirschblüten enthalten. Dieser Extrakt kann vorzugsweise auch die Öl-haltige Fraktion dieser Pflanzen umfassen.

Ganz besonders geeignet sind die Extrakte aus Moringa Olifeira, Grünem Tee, Mandel, Kokosnuss, Mango, Aprikose, Limone, Weizen, Kiwi und Melone.

Als Extraktionsmittel zur Herstellung der genannten Pflanzenextrakte können Wasser, Alkohole sowie deren Mischungen verwendet werden. Unter den Alkoholen sind dabei niedere Alkohole wie Ethanol und Isopropanol, insbesondere aber mehrwertige Alkohole wie Ethylenglykol und Propylenglykol, sowohl als alleiniges Extraktionsmittel als auch in Mischung mit Wasser, bevorzugt. Pflanzenextrakte auf Basis von Wasser/Propylenglykol im Verhältnis 1:10 bis 10:1 haben sich als besonders geeignet erwiesen.

Die Pflanzenextrakte können erfindungsgemäß sowohl in reiner als auch in verdünnter Form eingesetzt werden. Sofern sie in verdünnter Form eingesetzt werden, enthalten sie üblicherweise ca. 2 - 80 Gew.-% Aktivsubstanz und als Lösungsmittel das bei ihrer Gewinnung eingesetzte Extraktionsmittel oder Extraktionsmittelgemisch.

Weiterhin kann es bevorzugt sein, in den erfindungsgemäßen Zubereitungen Mischungen aus mehreren, insbesondere aus zwei, verschiedenen Pflanzenextrakten einzusetzen.

In einer weiteren bevorzugten Ausführungsform enthält das erfindungsgemäße Mittel mindestens ein Pflanzenöl. Erfindungsgemäß bevorzugte Ölpflanzen sind Soja, Baumwolle, Raps, Erdnuss, Sonnenblume, Palmkern, Kokosnuss, Walnuss, Olive, Haselnuss, Weintraube, Aprikose, Pfirsich, Orange, Zitrone, Distel, Kürbiskern, Mais, Nachtkerze, Hanf, Leinsaat, Mohn, Sesam und Weizenkeim. Der Einsatz von pflanzlichen Ölen in den erfindungsgemäßen Mittel ermöglicht eine überraschende Steigerung des Glanzes der behandelten Haare. Insbesondere die Öle aus Aprikose, Pfirsich und Nachtkerze zeichnen sich diesbezüglich aus.

Weiterhin ist es erfindungsgemäß bevorzugt 2-Pyrrolidinon-5-carbonsäure und deren Derivate als einzusetzen. Besonders bevorzugt sind die Natrium-, Kalium-, Calcium-, Magnesium- oder Ammoniumsalze, bei denen das Ammoniumion neben Wasserstoff eine bis drei C₁- bis C₄-Alkylgruppen trägt. Das Natriumsalz ist ganz besonders bevorzugt. Die eingesetzten Mengen in den erfindungsgemäßen Mitteln betragen 0,05 bis 10 Gew.-%, bezogen auf die gesamte Anwendungszubereitung, besonders bevorzugt 0,1 bis 5Gew.-%, und insbesondere 0,1 bis 3 Gew.-%.

Ferner konnten die Erfinder der vorliegenden Anmeldung zeigen, dass es sich vorteilhaft auf die Beschaffenheit des Schaumes auswirkt, wenn die Mittel frei von Silikonölen formuliert sind.

Als weiteren Bestandteil können die erfindungsgemäßen Zusammensetzungen mindestens eine Ammoniumverbindung aus der Gruppe Ammoniumchlorid, Ammoniumcarbonat, Ammoniumbicarbonat, Ammoniumsulfat und/oder Ammoniumcarbamat in einer Menge von 0,5 bis 10, vorzugsweise 1 bis 5 Gew.-%, bezogen auf die Gesamtzusammensetzung des Mittels enthalten.

Die erfindungsgemäßen Mittel können die Inhaltsstoffe in einem geeigneten wässrigen, alkoholischen oder wässrig-alkoholischen Träger enthalten. Es ist aber auch möglich, eine der beiden Komponenten als pulverförmige oder auch Tabletten-förmige Formulierung bereitzustellen.

Unter wässrig-alkoholischen Lösungen sind im Sinne der vorliegenden Erfindung wässrige Lösungen enthaltend 3 bis 70 Gew.-% eines C₁-C₄-Alkohols, insbesondere Ethanol bzw. Isopropanol, zu verstehen.

Die erfindungsgemäßen Mittel können erfindungsgemäß weitere organische Lösemittel, wie beispielsweise Monoethanolamin, Methoxybutanol, Benzylalkohol, Ethyldiglykol oder 1,2-Propylenglykol, enthalten. Bevorzugt sind dabei alle wasserlöslichen organischen Lösemittel. Monoethanolamin ist ein ganz besonders bevorzugtes organisches Lösemittel.

Bevorzugte erfindungsgemäße Mittel sind dadurch gekennzeichnet, dass sie zusätzlich ein nichtwässriges Lösungsmittel enthalten, wobei besonders bevorzugte erfindungsgemäße Mittel das Lösungsmittel in einer Konzentration von 0,1 - 30 Gewichtsprozent, bevorzugt in einer Konzentration von 1 - 20 Gewichtsprozent, ganz besonders bevorzugt in einer Konzentration von 2 - 10 Gewichtsprozent, jeweils bezogen auf das Mittel, enthalten.

In weiter bevorzugten erfindungsgemäßen Mitteln ist das Lösungsmittel ausgewählt aus Monoethanolamin, Ethanol, n-Propanol, Isoropanol, n-Butanol, Propylenglykol, n- Butylenglykol, Glycerin, Diethylenglykolmonoethylether, Diethylenglykolmono-n- butylether , Phenoxyethanol und Benzylalkohol sowie ihren Mischungen.

Die Formulierung in Wasser-basierten Lösungen, die frei von Fett- und Ölkörpern sind, hat sich erfindungsgemäß als besonders vorteilhaft erwiesen.

Der pH-Wert der erfindungsgemäßen Mittel kann durch geeignete Inhaltstoffe wie Acidifizierungsmittel oder Alkalisierungsmittel in einem weiten Bereich eingestellt werden.

In einer besonders bevorzugten Ausführungsform werden die erfindungsgemäßen Mittel dabei frei von Ammoniak als Alkalisierungsmittel formuliert. "Frei von Ammoniak" bedeutet dabei erfindungsgemäß, dass der Ammoniakgehalt der erfindungsgemäßen Mittel unterhalb von 1 Gew.-%, vorzugsweise unterhalb von 0,5 Gew.-%, insbesondere unterhalb von 0,1Gew.-%, jeweils bezogen auf das anwendungsbereite Mittel, liegt.

Weiterhin hat es sich als besonders bevorzugt erwiesen, wenn die resultierende Applikationsflüssigkeit dünnflüssig formuliert ist. Applikationsflüssigkeiten, die nach Mischung mit der Oxidationsmittelzubereitung eine Viskosität von 0 bis 2000 mPas aufweisen (gemessen bei 22°C im Brookfield-Viskosimeter Typ RV-T mit Spindel LV-1 beziehungsweise RV-1 und einer Geschwindigkeit von 30U/min), haben sich als besonders bevorzugt erwiesen. Eine Viskosität von 0 bis 1000mPas, gemessen unter den genannten Bedingungen, ist erfindungsgemäß besonders bevorzugt. Eine Viskosität von 5 bis 500 mPas, insbesondere von 10 bis 50 mPas (gemessen untern den oben genannten Bedingungen) ist erfindungsgemäß ganz besonders bevorzugt.

Es ist erfindungsgemäß bevorzugt, wenn die Mittel der vorliegenden Erfindung mittels einer speziellen Applikationsvorrichtung, wie beispielsweise einer mechanischen Schäumvorrichtung, aufgeschäumt werden. Unter mechanischen Schäumvorrichtungen sind solche Vorrichtungen zu verstehen, welche das Verschäumen einer Flüssigkeit mit oder ohne Verwendung eines Treibmittels ermöglichen. Erfindungsgemäß sind insbesondere derartige Schäumvorrichtungen bevorzugt, die eine Schaumbildung durch Luft aus der Umgebung und insbesondere ohne Verwendung von Treibgas ermöglichen.

Als geeignete mechanische Schäumvorrichtung kann beispielsweise eine Schüttelflasche, ein handelsüblicher Pumpschäumer oder ein so genannter Squeeze-schäumer verwendet werden.

Während in einer Schüttelflasche die Schaumbildung durch mechanische Agitation der Applikationsflüssigkeit innerhalb der Flasche stattfindet, weisen sowohl Pump- als auch Squeeze-Schäumer einen speziell ausgebildeten Schaumkopf auf. Ein derartiger Schaumkopf weist eine Schaumkammer auf, in die die Applikationszubereitung gemeinsam mit Umgebungsluft eingezogen und dabei aufgeschäumt wird. Während beim Pumpschäumer die Applikationsflüssigkeit über einen Pumpmechanismus eingezogen wird, erfolgt dies bei dem Squeezeschäumer durch manuellen Druck auf den Flaschenkörper. In allen Ausführungsformen hat es sich als erfindungsgemäß vorteilhaft erwiesen, wenn die Ausgabeeinheit eine Homogenisatoreinheit aufweist. Ein Beispiel für einen derartigen Homogenisator kann ein feinmaschiges Netz sein, das das bereits aufgeschäumte Produkt durchfließt. Aber auch andere Ausgestaltungsformen, wie beispielsweise poröse Sinterkörper können für diesen Zweck zum Einsatz kommen.

Erfindungsgemäß geeignete Pumpschäumer werden beispielsweise von der Firma Airspray unter der Handelsbezeichnung "Airfoamer" angeboten. Insbesondere bevorzugt ist der Airfoamer mit der Typenbezeichnung G3.

Auch Squeezefoamer sind prinzipiell bereits aus dem Stand der Technik bekannt. Erfindungsgemäß geeignete Schäumer sind beispielsweise von der Firma Supermatic Kunststoff AG in ihrem Internetauftritt www.squeeze-foamer.com beschrieben.

Die Anwendungstemperaturen des resultierenden Schaums können in einem Bereich zwischen 15 und 40 °C liegen. Nach einer Einwirkungszeit von 5 bis 45 Minuten wird das Haarfärbemittel durch Ausspülen von dem zu färbenden Haar entfernt.

Erfindungsgemäß bevorzugt ist auch der Einsatz von so genannten Komplexbildnern. Komplexbilder sind Stoffe, die Metallionen komplexieren können. Bevorzugte Komplexbildner sind sogenannte Chelatkomplexbildner, also Stoffe, die mit Metallionen cyclische Verbindungen bilden, wobei ein einzelner Ligand mehr als eine Koordinationsstelle an einem Zentralatom besetzt, d. h. mind. "zweizähnig" ist. In diesem Falle werden also normalerweise gestreckte Verbindungen durch Komplexbildung über ein Ion zu Ringen geschlossen. Die Zahl der gebundenen Liganden hängt von der Koordinationszahl des zentralen Ions ab.

Gebräuchliche und im Rahmen der vorliegenden Erfindung bevorzugte Chelatkomplexbilder sind beispielsweise Polyoxycarbonsäuren, Polyamine, Ethylendiamintetraessigsäure (EDTA), Nitrilotriessigsäure (NTA) und Hydroxyethandiphosphonsäuren bzw. deren Alkalisalze.

Im Rahmen der vorliegenden Erfindung können vorzugsweise Komplexbildner der folgenden chemischen Gruppen einzeln oder im Gemisch miteinander eingesetzt werden:
a) Polycarbonsäuren, bei denen die Summe der Carboxyl- und gegebenenfalls Hydroxylgruppen mindestens 5 beträgt wie Gluconsäure,
b) stickstoffhaltige Mono- oder Polycarbonsäuren wie Ethylendiamintetraessigsäure (EDTA), N-Hydroxyethylethylendiamintriessigsäure, Diethylentriaminpentaessigsäure, Hydroxyethyliminodiessigsäure, Nitridodiessigsäure-3-propionsäure, Isoserindiessigsäure, N,N-Di-(β-hydroxyethyl)glycin, N-(1,2-Dicarboxy-2-hydroxyethyl)-glycin, N-(1,2-Dicarboxy-2-hydroxyethyl)asparaginsäure oder Nitrilotriessigsäure (NTA),
c) geminale Diphosphonsäuren wie 1-Hydroxyethan-1,1-diphosphonsäure (HEDP), deren höhere Homologe mit bis zu 8 Kohlenstoffatomen sowie Hydroxy- oder Aminogruppen-haltige Derivate hiervon und 1-Aminoethan-1,1-diphosphonsäure, deren höhere Homologe mit bis zu 8 Kohlenstoffatomen sowie Hydroxy- oder Aminogruppen-haltige Derivate hiervon,

Als Polycarbonsäuren a) werden im Rahmen dieser Patentanmeldung Carbonsäuren -auch Monocarbonsäuren- verstanden, bei denen die Summe aus Carboxyl- und den im Molekül enthaltenen Hydroxylgruppen mindestens 5 beträgt. Komplexbildner aus der Gruppe der stickstoffhaltigen Polycarbonsäuren, insbesondere EDTA, sind bevorzugt. Bei den erfindungsgemäß erforderlichen alkalischen pH-Werten der Behandlungslösungen liegen diese Komplexbildner zumindest teilweise als Anionen vor. Es ist unwesentlich, ob sie in Form der Säuren oder in Form von Salzen eingebracht werden. Im Falle des Einsatzes als Salze sind Alkali-, Ammonium- oder Alkylammoniumsalze, insbesondere Natriumsalze, bevorzugt.

Eine weitere Substanzklasse mit komplexbildenden Eigenschaften stellen die Phosphonate dar. Dabei handelt es sich insbesondere um Hydroxyalkan- bzw. Aminoalkanphosphonate. Unter den Hydroxyalkanphosphonaten ist das 1-Hydroxyethan-1,1-diphosphonat (HEDP) von besonderer Bedeutung. Es wird vorzugsweise als Natriumsalz eingesetzt, wobei das Dinatriumsalz neutral und das Tetranatriumsalz alkalisch (pH 9) reagiert. Als Aminoalkanphosphonate kommen vorzugsweise Ethylendiamintetramethylenphosphonat (EDTMP), Diethylentriaminpentamethylenphosphonat (DTPMP) sowie deren höhere Homologe in Frage. Sie werden vorzugsweise in Form der neutral reagierenden Natriumsalze, z. B. als Hexanatriumsalz der EDTMP bzw. als Hepta- und OctaNatriumsalz der DTPMP, eingesetzt. Als Komplexbildner wird dabei aus der Klasse der Phosphonate bevorzugt HEDP verwendet. Die Aminoalkanphosphonate besitzen zudem ein ausgeprägtes Schwermetallbindevermögen. Dementsprechend kann es, insbesondere wenn die Mittel auch Bleiche enthalten, bevorzugt sein, Aminoalkanphosphonate, insbesondere DTPMP, einzusetzen, oder Mischungen aus den genannten Phosphonaten zu verwenden. Diese Stoffe werden nachstehend beschrieben.

Erfindungsgemäß bevorzugte Komplexbildner sind Phosphonate, vorzugsweise Hydroxyalkan- bzw. Aminoalkanphosphonate und insbesondere 1-Hydroxyethan-1,1-diphosphonat (HEDP) bzw. dessen Di- oder Tetranatriumsalz und/oder Ethylendiamintetramethylenphosphonat (EDTMP) bzw. dessen Hexanatriumsalz und/oder Diethylentriaminpentamethylenphosphonat (DTPMP) bzw. dessen Hepta- oder Octanatriumsalz.

Auch Dipicolinsäure wird erfindungsgemäß vorzugsweise als Komplexbildner eingesetzt. Mittel, die eine Kombination aus einem EDTA-Salz und, HEDP und Dipicolinsäure enthalten sind erfindungsgemäß ganz besonders bevorzugt.

Ein zweiter Gegenstand der vorliegenden Erfindung ist ein Verfahren zur Färbung keratinischer Fasern, bei dem drei getrennt voneinander konfektionierte Mittel A, B und C bereitgestellt werden, wobei
a. das erste Mittel (A) tensidfrei ist und mindestens ein Oxidationsfarbstoffvorprodukt enthält und
b. das zweite Mittel (B) tensidfrei ist und mindestens ein Oxidationsmittel enthält und
c. das dritte Mittel (C) mindestens ein Tensid enthält und
d. mindestens eines der Mittel A, B oder C in einer Applikationsvorrichtung enthalten ist, die geeignet ist, die Mittel A, B und C miteinander zu mischen und die Mischung in Form eines Schaumes abzugeben,
die Mittel A, B und C in der Applikationsvorrichtung miteinander vermischt und die Mischung aus dieser Applikationsvorrichtung als Schaum ausgebracht wird, dieser auf den Fasern verteilt wird, dann für eine Zeit von 1 bis 60 Minuten, vorzugsweise von 5 bis 40 Minuten, auf den Fasern verbleibt und anschließend aus den Fasern ausgewaschen wird.

Dabei kann der ausgebrachte Schaum unmittelbar auf den Haaransatz aufgetragen und anschließend mit den Händen oder einem mechanischen Hilfsmittel auf den Fasern verteilt werden. Es ist aber auch denkbar, dass der Schaum zunächst auf ein mechanisches Hilfsmittel, wie beispielsweise einen Kamm, aufgebracht wird und anschließend mit dessen Hilfe auf den Fasern verteilt wird. Unabhängig von der Art der Auftragung kann es erfindungsgemäß bevorzugt sein, wenn der Schaum anschließend in die Haare einmassiert wird.

Mindestens eines der Mittel A, B oder C ist im Rahmen des erfindungsgemäßen Verfahrens in einer Applikationsvorrichtung enthalten ist, die geeignet ist, die Mittel A, B und C miteinander zu mischen und die Mischung in Form eines Schaumes abzugeben. Vorzugsweise handelt es sich hierbei um das Mittel A oder das Mittel B, um das nachträgliche Hinzufügen des Tensids zu ermöglichen. Ganz besonders bevorzugt ist das Mittel B (die tensidfreie Oxidationsmittelzubereitung) in einer Applikationsvorrichtung enthalten, die geeignet ist, die Mittel A, B und C miteinander zu mischen und die Mischung in Form eines Schaumes abzugeben.

Die beiden anderen Mittel (in der ganz besonders bevrozugten Verfahrensvariante also die Mittel A und C) werden dann zu dem Mittel in der Applikationsvorrichtung gegeben, die Applikationsvorrichtung wieder verschlossen und die Mittel durch Schütteln oder Schwenken miteinander vermischt. Es hat sich als besonders bevorzugt erwiesen, zunächst die Mittel A und B miteinander vorzumischen und die Mischung aus den Mitteln A und B in der Applikationsvorrichtung anschließend mit dem Mittel C zu versetzen und erneut zu mischen. Hierdurch wird ein besonders stabiler, cremiger und gut applizierbarer Schaum erhalten und zudem das risiko des Überschäumens beim Mischen vor dem verschließen des Applikationsbehältnisses vermieden.

Besonders bevorzugte erfindungsgemäße Verfahren sind dadurch gekennzeichnet, daß zunächst die Mittel A und B in der Applikationsvorrichtung miteinander vermischt werden, dann Mittel C hinzugefügt und mit der Mischung aus Mittel A und B vermischt und die Mischung aus dieser Applikationsvorrichtung als Schaum ausgebracht wird.

Nach der Mischung aller drei Mittel in der Applikatiionsvorrichtung sollte der Schaum alsbald äppliziert werden, um ein optimales Frbeergebnis zu erzielen. Hier sind erfindungsgemäße Verfahren bevorzugt, bei denen die Mischung der Mittel A, B und C innerhalb eines Zeitraumes von 0 bis 30 Minuten nach dem Mischvorgang aus der Applikationsvorrichtung als Schaum ausgebracht wird.

Bezüglich weiterer bevorzugter Varianten des erfindungsgemäßen Verfahrens gilt mutatis mutandis das zu den erfindungsgemäßen Kits Gesagte.

Besonders bevorzugt ist es, die Ausfärbung durch physikalische Maßnahmen zu unterstützen. Erfindungsgemäße Verfahren, bei denen die Anwendung durch Einwirkung von Wärme und/oder UV-Strahlung während der Einwirkzeit unterstützt wird.

## Patentansprüche

1. Haarbehandlungs-Kit, umfassend mindestens drei getrennt voneinander konfektionierte Mittel A, B und C, **dadurch gekennzeichnet, dass**
a. das erste Mittel (A) tensidfrei ist und mindestens ein Oxidationsfarbstoffvorprodukt enthält und
b. das zweite Mittel (B) tensidfrei ist und mindestens ein Oxidationsmittel enthält und
c. das dritte Mittel (C) mindestens ein Tensid enthält und
d. mindestens eines der Mittel A, B oder C in einer Applikationsvorrichtung enthalten ist, die geeignet ist, die Mittel A, B und C miteinander zu mischen und die Mischung in Form eines Schaumes abzugeben, wobei die Applikationsvorrichtung ein Pumpschäumer oder ein Squeezeschäumer ist.

2. Haarbehandlungs-Kit nach Anspruch 1, **dadurch gekennzeichnet, dass** das erste Mittel (A), bezogen auf das Gewicht des Mittels A, 0,1 bis 20 Gew.-%, vorzugsweise 0,25 bis 17,5 Gew.-%, weiter bevorzugt 0,5 bis 15 und insbesondere 0,75 bis 10 Gew.-% Oxidationsfarbstoffvorprodukt(e) enthält.

3. Haarbehandlungs-Kit nach einem der Ansprüche 1 oder 2, **dadurch gekennzeichnet, dass** das erste Mittel (A) zusätzlich mindestens einen direktziehenden Farbstoff enthält.

4. Haarbehandlungs-Kit nach einem der Ansprüche 1 bis 3, **dadurch gekennzeichnet, dass** das zweite Mittel (B), bezogen auf das Gewicht des Mittels B, 0,5 bis 20 Gew.-%, vorzugsweise 1,0 bis 17,5 Gew.-%, weiter bevorzugt 2,5 bis 16 Gew.-% und insbesondere 5 bis 14 Gew.-% Wasserstoffperoxid (berechnet als 100 %iges H₂O₂) enthält.

5. Haarbehandlungs-Kit nach einem der Ansprüche 1 bis 4, **dadurch gekennzeichnet, dass** das dritte Mittel (C), bezogen auf das Gewicht des Mittels C, 2,5 bis 100 Gew.-%, vorzugsweise 5 bis 95 Gew.-%, weiter bevorzugt 7,5 bis 90 Gew.-% und insbesondere 10 bis 75 Gew.-% Tensid(e) enthält.

6. Haarbehandlungs-Kit nach einem der Ansprüche 1 bis 5, **dadurch gekennzeichnet, dass** die Mischung der Mittel A, B und C bezogen auf das Gewicht der Mischung mindestens 10,5 Gew.-%, vorzugsweise mindestens 11 Gew.-%, weiter bevorzugt mindestens 12 Gew.-%, noch weiter bevorzugt mindestens 13 Gew.-% und insbesondere mindestens 14 Gew.-% Tensid(e) enthält.

7. Verfahren zur Färbung keratinischer Fasern, **dadurch gekennzeichnet, dass** drei getrennt voneinander konfektionierte Mittel A, B und C bereitgestellt werden, wobei
a. das erste Mittel (A) tensidfrei ist und mindestens ein Oxidationsfarbstoffvorprodukt enthält und
b. das zweite Mittel (B) tensidfrei ist und mindestens ein Oxidationsmittel enthält und
c. das dritte Mittel (C) mindestens ein Tensid enthält und
d. mindestens eines der Mittel A, B oder C in einer Applikationsvorrichtung enthalten ist, die geeignet ist, die Mittel A, B und C miteinander zu mischen und die Mischung in Form eines Schaumes abzugeben,
die Mittel A, B und C in der Applikationsvorrichtung miteinander vermischt und die Mischung aus dieser Applikationsvorrichtung als Schaum ausgebracht wird, dieser auf den Fasern verteilt wird, dann für eine Zeit von 1 bis 60 Minuten, vorzugsweise von 5 bis 40 Minuten, auf den Fasern verbleibt und anschließend aus den Fasern ausgewaschen wird.

8. Verfahren nach Anspruch 7, **dadurch gekennzeichnet, dass** die Mischung der Mittel A, B und C innerhalb eines Zeitraumes von 0 bis 30 Minuten nach dem Mischvorgang aus der Applikationsvorrichtung als Schaum ausgebracht wird.

9. Verfahren nach einem der Ansprüche 7 oder 8, **dadurch gekennzeichnet, dass** zunächst die Mittel A und B in der Applikationsvorrichtung miteinander vermischt werden, dann Mittel C hinzugefügt und mit der Mischung aus Mittel A und B vermischt und die Mischung aus dieser Applikationsvorrichtung als Schaum ausgebracht wird.

## Claims

1. A hair-treatment kit, comprising at least three separately packaged agents A, B and C, **characterized in that**
a. the first agent (A) is surfactant-free and contains at least one oxidation dye precursor, and
b. the second agent (B) is surfactant-free and contains at least one oxidizing agent, and
c. the third agent (C) contains at least one surfactant, and
d. at least one of the agents A, B or C is contained in an application device that is suitable for mixing together the agents A, B and C and dispensing the mixture in the form of a foam, the application device being a pump foamer or a squeeze foamer.

2. The hair-treatment kit according to claim 1, **characterized in that** the first agent (A) contains, based on the weight of the agent A, 0.1 to 20 wt.%, preferably 0.25 to 17.5 wt.%, more preferably 0.5 to 15 wt.% and in particular 0.75 to 10 wt.% oxidation dye precursor(s).

3. The hair-treatment kit according to one of claims 1 or 2, **characterized in that** the first agent (A) additionally contains at least one direct dye.

4. The hair-treatment kit according to one of claims 1 to 3, **characterized in that** the second agent (B) contains, based on the weight of the agent B, 0.5 to 20 wt.%, preferably 1.0 to 17.5 wt.%, more preferably 2.5 to 16 wt.% and in particular 5 to 14 wt.% hydrogen peroxide (calculated as 100% H₂O₂).

5. The hair-treatment kit according to one of claims 1 to 4, **characterized in that** the third agent (C) contains, based on the weight of the agent C, 2.5 to 100 wt.%, preferably 5 to 95 wt.%, more preferably 7.5 to 90 wt.% and in particular 10 to 75 wt.% surfactant(s).

6. The hair-treatment kit according to one of claims 1 to 5, **characterized in that** the mixture of agents A, B and C contains, based on the weight of the mixture, at least 10.5 wt.%, preferably at least 11 wt.%, more preferably at least 12 wt.%, even more preferably at least 13 wt.% and in particular at least 14 wt.% surfactant(s).

7. A method for coloring keratin fibers, **characterized in that** three separately packaged agents A, B and C are provided,
a. the first agent (A) being surfactant-free and containing at least one oxidation dye precursor, and
b. the second agent (B) being surfactant-free and containing at least one oxidizing agent, and
c. the third agent (C) containing at least one surfactant, and
d. at least one of the agents A, B or C being contained in an application device that is suitable for mixing together the agents A, B and C and dispensing the mixture in the form of a foam,
the agents A, B and C being mixed together in the application device and the mixture being dispensed from said application device as a foam, said foam being distributed over the fibers, then remaining on the fibers for a period of from 1 to 60 minutes, preferably 5 to 40 minutes, and subsequently being washed out of the fibers.

8. The method according to claim 7, **characterized in that** the mixture of the agents A, B and C is dispensed from the application device as a foam within a period of from 0 to 30 minutes after the mixing process.

9. The method according to one of claims 7 or 8, **characterized in that** the agents A and B are initially mixed together in the application device, then agent C is added and mixed with the mixture of agents A and B, and the mixture is dispensed from said application device as a foam.

## Revendications

1. Kit de traitement capillaire comprenant au moins trois agents A, B et C confectionnés séparément, **caractérisé en ce que**
a. le premier agent (A) est exempt de tensioactif et contient au moins un précurseur de colorant d'oxydation et
b. le deuxième agent (B) est exempt de tensioactif et contient au moins un agent oxydant et
c. le troisième agent (C) contient au moins un tensioactif et
d. au moins un des agents A, B ou C est contenu dans un dispositif d'application qui est approprié pour mélanger les agents A, B et C entre eux et délivrer le mélange sous la forme d'une mousse, le dispositif d'application étant un générateur de mousse par pompage ou un générateur de mousse par pressage.

2. Kit de traitement capillaire selon la revendication 1, **caractérisé en ce que** le premier agent (A) contient, sur la base du poids de l'agent A, de 0,1 à 20% en poids, de préférence de 0,25 à 17,5% en poids, plus préférablement de 0,5 à 15% en poids et en particulier de 0,75 à 10% en poids d'un ou de plusieurs précurseur de colorant d'oxydation (e).

3. Kit de traitement capillaire selon l'une des revendications 1 ou 2, **caractérisé en ce que** le premier agent (A) contient en outre au moins un colorant direct.

4. Kit de traitement capillaire selon l'une des revendications 1 à 3, **caractérisé en ce que** le deuxième agent (B) contient, par rapport au poids de l'agent B, de 0,5 à 20% en poids, de préférence de 1,0 à 17,5% en poids, plus préférablement de 2,5 à 16% en poids et en particulier de 5 à 14% en poids de peroxyde d'hydrogène (calculé sous forme de H₂O₂ à 100%).

5. Kit de traitement capillaire selon l'une des revendications 1 à 4, **caractérisé en ce que** le troisième agent (C) contient, par rapport au poids de l'agent C, de 2,5 à 100% en poids, de préférence de 5 à 95% en poids, plus préférablement de 7,5 à 90% en poids et en particulier de 10 à 75% en poids d'un ou de plusieurs tensioactifs.

6. Kit de traitement capillaire selon l'une des revendications 1 à 5, **caractérisé en ce que** le mélange des agents A, B et C contient, sur la base du poids du mélange, au moins 10,5% en poids, de préférence au moins 11 % en poids, plus préférablement au moins 12% en poids, encore plus préférablement au moins 13% en poids et en particulier au moins 14% en poids d'un ou de plusieurs tensioactifs.

7. Procédé de coloration de fibres de kératine, **caractérisé en ce que** trois agents A, B et C confectionnés séparément sont produits,
a. le premier agent (A) est exempt de tensioactif et contient au moins un précurseur de colorant d'oxydation et
b. le deuxième agent (B) est exempt de tensioactif et contient au moins un agent oxydant et
c. le troisième agent (C) contient au moins un tensioactif et
d. au moins un des agents A, B ou C est contenu dans un dispositif d'application qui est approprié pour mélanger les agents A, B et C entre eux et délivrer le mélange sous la forme d'une mousse,
les agents A, B et C étant mélangés entre eux dans le dispositif d'application et le mélange étant délivrée en sortie de ce dispositif d'application sous la forme d'une mousse, celle-ci étant répartie sur les fibres, puis restant sur les fibres pendant une durée de 1 à 60 minutes, de préférence de 5 à 40 minutes, et étant ensuite éliminée des fibres par lavage.

8. Procédé selon la revendication 7, **caractérisé en ce que** le mélange des agents A, B et C étant délivrés en sortie du dispositif d'application sous la forme d'une mousse au bout de 0 à 30 minutes après l'opération de mélange.

9. Procédé selon l'une des revendications 7 ou 8, **caractérisé en ce que** les agents A et B sont mélangés entre eux dans le dispositif d'application, puis l'agent C est ajouté et mélangé au mélange des agents A et B, et le mélange est délivré en sortie de ce dispositif d'application sous la forme d'une mousse.
